# EUROPEAN PATENT SPECIFICATION

(11) **EP 4 274 624 B1**
(45) Date of publication and mention of the grant of the patent: **25.09.2024**
(21) Application number: 22700016.3
(22) Date of filing: 03.01.2022
(51) Int. Cl.: A61L 26/00, A61L 24/00

(54) **BIORESORBABLE SEALING POWDER**
BIORESORBIERBARES DICHTUNGSPULVER
POUDRE D'ÉTANCHÉITÉ BIORÉSORBABLE

(30) Priority: 08.01.2021 EP 21150755
(43) Date of publication of application: 15.11.2023
(60) Divisional application: 24194806.6
(73) Proprietor: Cilag GmbH International, 6300 Zug (CH)
(72) Inventor: BENDER, Johannes Caspar Mathias Elizabeth, 6534 AT Nijmegen (NL); VAN DIJCK, Julius Marcus, 6534 AT Nijmegen (NL)
(74) Representative: Nederlandsch Octrooibureau
(86) International application number: PCT/EP2022/050024
(87) International publication number: WO 2022/148725

(56) References cited:
- WO-A1-2013/137736
- WO-A1-2016/056901
- WO-A2-2012/057628

## Description

### TECHNICAL FIELD OF THE INVENTION

The present invention relates to a bioresorbable sealing powder comprising:
(a) water-soluble electrophilic polymer carrying at least 3 reactive electrophilic groups that are capable of reacting with amine groups under the formation of a covalent bond;
(b) water-soluble nucleophilic cross-linker carrying at least 2 reactive nucleophilic groups that, in the presence of water, are capable of reacting with the reactive electrophilic groups of the electrophilic polymer under the formation of a covalent bond between the electrophilic polymer and the nucleophilic cross-linker;
(c) water-absorbing particles containing at least 50% by weight of said water-absorbing particles of water-insoluble polymer containing reactive nucleophilic groups selected from amine groups, thiol groups;
(d) water-soluble dispersant that is solid at 20°C, said water-soluble dispersant being selected from monosaccharides, disaccharides, oligosaccharides, sugar alcohols and combinations thereof;
wherein the components (a), (b), (c) and (d) may be contained in the same particles or in different particles.

When the sealing powder of the present invention is applied onto wet tissue, the reactive components in the powder quickly react under the formation of a tissue-adhesive hydrogel that seals off the underlying tissue. The adhesive hydrogel provides a seal that is strong enough to provide an effective seal to lung air leaks.

### BACKGROUND OF THE INVENTION

Hemostasis is a tightly regulated process that maintains the blood flow through the vasculature simultaneously as a thrombotic response to tissue damage occurs. Maintaining hemostasis requires a complex interaction of the vessel wall, platelets, and the coagulation and fibrinolytic systems. There are two main phases of hemostasis: primary (i.e., the cellular phase) and secondary (i.e., the humoral phase).

Primary hemostasis begins immediately after endothelial disruption and is characterized by vasoconstriction, platelet adhesion, and formation of a soft aggregate plug. After the injury occurs, there is a temporary local contraction of vascular smooth muscle and the blood flow slows, promoting platelet adhesion and activation. Within 20 seconds of the injury, circulating von Willebrand factor attaches to the subendothelium at the site of injury and adheres to the glycoproteins on the surface of platelets. As platelets adhere to the injured surface, they are activated by contact with collagen-exposing receptors that bind circulating fibrinogen. A soft plug of aggregated platelets and fibrinogen is formed. This phase of hemostasis is short lived, and the soft plug can easily be sheared from the injured surface.

The soft platelet plug is stabilized during secondary hemostasis to form a clot. Vasoconstriction and the resultant reduction in blood flow are maintained by platelet secretion of serotonin, prostaglandin, and thromboxane while the coagulation cascade is initiated. The coagulation cascade is a series of dependent reactions involving several plasma proteins, calcium ions, and blood platelets that lead to the conversion of fibrinogen to fibrin. Coagulation factors are produced by the liver and circulate in an inactive form until the coagulation cascade is initiated. Then each step of the cascade is initiated and completed via a series of sequential and dependent coagulation factor activation reactions. In the final steps, thrombin converts the soluble plasma protein fibrinogen to the insoluble protein fibrin, while simultaneously converting factor XIII to factor Xllla. This factor conversion stabilizes the fibrin and results in cross-linking of the fibrin monomers, producing a stable clot. During surgery, it is important to maintain a fine balance between bleeding and clotting, such that blood continues to flow to the tissues at the surgical site without excessive loss of blood, to optimize surgical success and patient outcome. Continuous bleeding from diffuse minor capillaries or small venules during surgery can obscure the surgical field, prolong operating time, increase the risk of physiologic complications, and expose the patient to risks associated with blood transfusion.

Surgeons have an array of options to control bleeding, including mechanical and thermal techniques and devices as well as pharmacotherapies and topical agents.

One of the earliest topical hemostatic agents was cotton, in the form of gauze sponges. Although such materials concentrate blood and coagulation products via physical adsorption, they are not absorbed by the body, and upon removal, the clot may be dislodged, leading to further bleeding. Absorbable topical hemostatic agents have since been developed and provide useful adjunctive therapy when conventional methods of hemostasis are ineffective or impractical. Topical hemostatic agents can be applied directly to the bleeding site and may prevent continuous unrelenting bleeding. Hemostasis using topical agents also can avoid the adverse effects of systemic hemostatic medications, such as "unwanted" blood clots. Furthermore, in surgical procedures where the amount of blood loss is unpredictable, topical hemostats can be used sparingly when blood loss is minimal and more liberally during severe bleeding.

A number of topical hemostatic agents are currently available for use in surgery. They can be divided into two categories: those that provide their mechanism of action on the clotting cascade in a biologically active manner and those that act passively through contact activation and promotion of platelet aggregation. Passive topical hemostatic agents include collagens, cellulose, and gelatins, while active agents include thrombin and products in which thrombin is combined with a passive agent to provide an active overall product.

US 2003/0064109 describes a dry hemostatic powder that is prepared by a method comprising: providing an aqueous solution comprising gelatin combined with at least one re-hydration aid; drying the solution to produce solids; grinding the solids to produce a powder; cross-linking the powder; removing at least 50% (w/w) of the re-hydration aid; and drying the cross-linked gelatin to produce a powder. The re-hydration aid may comprise at least one material selected from the group consisting of polyethylene glycol (PEG), polyvinylpyrrolidone (PVP), and dextran.

US 2012/0021058 describes a process for making a hemostatic composition, said process comprising: a) providing a dry granular preparation of a biocompatible polymer; and b) coating the granules in said dry granular preparation with a preparation of a coagulation inducing agent, such as a thrombin solution. The biocompatible polymer may be selected from of gelatin, soluble collagen, albumin, hemoglobin, fibrinogen, fibrin, casein, fibronectin, elastin, keratin, laminin, and derivatives or combinations thereof.

US 2013/0316974 describes a hemostatic material comprising a compacted ORC powder comprising particles having average aspect ratio from about 1 to about 18. The hemostatic material may further comprise an additive selected from polysaccharides, calcium salt, anti-infective agent, hemostasis promoting agent, gelatin, collagen.

US 2016/0271228 describes a hemostatic composition comprising:
- a hemostatic biocompatible polymer in particulate form selected from the group consisting of a protein, a polysaccharide, a biologic polymer, a non-biologic polymer, and derivatives and combinations thereof, wherein the hemostatic biocompatible polymer in particulate form is present as granular particles having a median diameter range of 50-700 µm;
- one hydrophilic cross-linker comprising electrophilic reactive groups, wherein the electrophilic reactive groups retain their reactivity until the composition is exposed to blood of the patient, wherein the electrophilic reactive groups are configured to cross-link with blood proteins of the patient to form a gel with sealing and hemostatic properties; and
- a binder that does not react with the electrophilic reactive groups of the one hydrophilic cross-linker;
wherein the hemostatic composition is in paste form.

WO 2012/057628 describes a kit for producing a biocompatible, cross-linked polymer, said kit comprising an electrophilically activated polyoxazoline (EL-POx), said EL-POX comprising m electrophilic groups; and said nucleophilic cross-linking agent comprising n nucleophilic groups, wherein the m electrophilic groups are capable of reaction with the n nucleophilic groups to form covalent bonds; wherein m>2, n>2 and m+n >5; wherein at least one of the m electrophilic groups is a pendant electrophilic group.

WO 2016/056901 describes an adhesive hemostatic product selected from a coated mesh, a coated foam or a coated powder, said hemostatic product comprising:
- a porous solid substrate having a porosity of at least 5 vol.% and comprising an outer surface that comprises a nucleophilic polymer containing reactive nucleophilic groups;
- an adhesive coating that covers at least a part of the solid substrate, said coating comprising an electrophilically activated polyoxazoline (EL-POX), said EL-POX containing on average at least 1 reactive electrophilic group.

US 2016/0375202 describes a device for expression of a hemostatic powder, comprising:
a) an elongated hollow reservoir, the reservoir having a manual air pump attached to the reservoir and an expression port at a distal end of said reservoir,
b) a porous filter slidably disposed within the reservoir between said air pump and said expression port;
c) a spring disposed within the reservoir between the air pump and the filter;
wherein the powder is disposed within the reservoir between the filter and the expression port, and the pump is in a fluid communication with the expression port through the porous filter and through the powder.

### SUMMARY OF THE INVENTION

The inventors have developed a bioresorbable sealing powder that can conveniently be used to control bleeding during surgery and/or to provide a protective seal.

The sealing powder of the present invention comprises
(a) at least 5 wt.% of water-soluble electrophilic polymer carrying at least 3 reactive electrophilic groups that are capable of reacting with amine groups under the formation of a covalent bond;
(b) 1-50 wt.% of water-soluble nucleophilic cross-linker carrying at least 2 reactive nucleophilic groups that, in the presence of water, are capable of reacting with the reactive electrophilic groups of the electrophilic polymer under the formation of a covalent bond between the electrophilic polymer and the nucleophilic cross-linker;
(c) 1-60 wt.% of water-absorbing particles containing at least 50% by weight of said water-absorbing particles of water-insoluble polymer containing reactive nucleophilic groups selected from amine groups, thiol groups and combinations thereof;
(d) 10-75 wt.% of water-soluble dispersant that is solid at 20°C, said water-soluble dispersant being selected from monosaccharides, disaccharides, oligosaccharides, sugar alcohols and combinations thereof;

wherein the combination of components (a), (b), (c) and (d) constitutes at least 60 wt.% of the sealing powder;
wherein the sealing powder has a tapped density in the range of 0.3 - 0.9 g/ml;
wherein at least 90 wt.% of the powder has a diameter below 600 µm and not more than 10 wt.% of the powder has a diameter below 10 µm; and
wherein the components (a), (b), (c) and (d) may be contained in the same particles or in different particles.

When applied onto wet tissue, the sealing powder of the present invention quickly forms a seal in the form of a hydrogel that sticks to the tissue. The sealing powder may suitably be used to provide an effective seal to e.g. lung air leaks. In addition, the sealing powder has excellent hemostatic capacity due to the fact that it is capable of absorbing large quantities of blood under the formation of strongly gelled blood clot that seals off the bleeding site.

The sealing powder of the present invention can easily be distributed over a tissue. If necessary, additional sealing powder can be applied to form an additional sealing layer that sticks to the underlying layer of hydrogel.

Although the inventors do not wish to be bound by theory, it is believed that when a layer of the hemostatic powder is applied onto wet tissue, the water-soluble electrophilic polymer and the water-soluble nucleophilic polymer rapidly dissolve. The dissolved electrophilic polymer reacts with reactive nucleophilic groups of dissolved water-soluble nucleophilic polymer under the formation of a hydrogel in which the water-absorbing particles are entrained. These water-absorbing particles provide moisture absorbing capacity due to their capability to swell. The dissolved electrophilic polymer also reacts with proteins in tissue, thereby fixating the hydrogel to the tissue. Furthermore, the dissolved electrophilic polymer reacts with proteins in blood, thereby producing a gelled blood clot.

The water-soluble dispersant in the sealing powder of the present invention ensures that the other components of the sealing powder are rapidly and evenly dispersed when the sealing powder comes into contact with moisture. Furthermore, the inclusion of water-soluble dispersant enables the preparation of a sealing powder that is composed of particles that have a sufficiently high density to enable accurate application of the powder onto tissue by means of air flow, e.g. air flow generated by a bellows.

Surprisingly, after the sealing powder has been applied onto wet tissue, it can suitably be pressed with a saline soaked wet gauze pad as the gauze pad does not stick to the gelled/gelling sealing powder.

Another aspect of the invention relates to a method of preparing the bioresorbable sealing powder of the present invention, comprising the steps of:
(a) providing particles A comprising the electrophilic polymer and the water-soluble dispersant;
(b) providing particles B comprising the nucleophilic cross-linker, the water-absorbing particles and the water-soluble dispersant;
(c) combining the particles A and particles B.

A further aspect of the invention relates to a device for powder application comprising:
- a reservoir containing the bioresorbable sealing powder of the present invention;
- an elongated hollow tubular structure having a proximal and a distal end, the distal end having a powder outlet, the proximal end being connected to the reservoir,
- a manual air pump, preferably a bulb or bellows, arranged to produce an air flow that carries powder from the reservoir via the elongated hollow tubular structure through the powder outlet.

Another aspect of the invention relates to a biocompatible, flexible, hemostatic sheet comprising:
- a cohesive fibrous carrier structure comprising a three-dimensional interconnected interstitial space; and
- the bioresorbable sealing powder of the present invention;
wherein the sealing powder is distributed within the interstitial space and/or fixated onto the fibrous carrier structure.

The invention also relates to a kit of parts for preparing a bioresorbable sealing suspension, said kit comprising:
- a first container or compartment comprising a biocompatible liquid; and
- a second container or compartment comprising the bioresorbable sealing powder of the present invention.

Yet another aspect of the invention relates to a bioresorbable sealing suspension comprising:
- a biocompatible continuous liquid non-aqueous phase; and
- a dispersed phase comprising the bioresorbable sealing powder of the present invention.

### DETAILED DESCRIPTION OF THE INVENTION

Accordingly, a first aspect of the invention relates to a bioresorbable sealing powder comprising:
(a) at least 5 wt.% of water-soluble electrophilic polymer carrying at least 3 reactive electrophilic groups that are capable of reacting with amine groups under the formation of a covalent bond;
(b) 1-50 wt.% of water-soluble nucleophilic cross-linker carrying at least 2 reactive nucleophilic groups that, in the presence of water, are capable of reacting with the reactive electrophilic groups of the electrophilic polymer under the formation of a covalent bond between the electrophilic polymer and the nucleophilic cross-linker;
(c) 1-60 wt.% of water-absorbing particles containing at least 50% by weight of said water-absorbing particles of water-insoluble polymer containing reactive nucleophilic groups selected from amine groups, thiol groups and combinations thereof;
(d) 10-75 wt.% of water-soluble dispersant that is solid at 20°C, said water-soluble dispersant being selected from monosaccharides, disaccharides, oligosaccharides, sugar alcohols and combinations thereof;

wherein the combination of components (a), (b), (c) and (d) constitutes at least 60 wt.% of the sealing powder;
wherein the sealing powder has a tapped density in the range of 0.3 - 0.9 g/ml;
wherein at least 90 wt.% of the powder has a diameter below 600 µm and not more than 10 wt.% of the powder has a diameter below 10 µm; and
wherein the components (a), (b), (c) and (d) may be contained in the same particles or in different particles.

The term "bioresorbable sealing powder" as used herein means that all the components of the sealing powder are resorbed by the body. Some of the components of the sealing powder, notably polymeric components, are gradually degraded in the body before being resorbed.

The term "water-soluble electrophilic polymer" as used herein refers to an electrophilic polymer that has a solubility in demineralized water of 20°C, at pH 7, of at least 50 g/L. In order to determine water solubility of the nucleophilic polymer at different pH, the pH of the demineralized water is adjusted using hydrochloric acid.

The term "polyoxazoline" as used herein refers to a poly(N-acylalkylenimine) or a poly(aroylalkylenimine) and is further referred to as POx. An example of POx is poly(2-ethyl-2-oxazoline). The term "polyoxazoline" also encompasses POx copolymers.

The term "water-soluble nucleophilic cross-linker" as used herein refers to a nucleophilic cross-linker that has a solubility in demineralized water of 20°C, at pH 7, of at least 50 g/L. In order to determine water solubility of the nucleophilic cross-linker at different pH, the pH of the demineralized water is adjusted using hydrochloric acid.

The term "protein" as used herein, unless indicated otherwise, also encompasses cross-linked and hydrolysed proteins. Likewise, unless indicated otherwise, whenever reference is made to a particular protein species, such as gelatin or collagen, also hydrolysed and cross-linked versions of that protein species are encompassed.

The term "collagen" as used herein refers to the main structural protein in the extracellular space of various connective tissues in animal bodies. Collagen forms a characteristic triple helix of three polypeptide chains. Depending upon the degree of mineralization, collagen tissues may be either rigid (bone) or compliant (tendon) or have a gradient from rigid to compliant (cartilage). Unless indicated otherwise, the term "collagen" also encompasses modified collagens other than gelatin (e.g. cross-linked collagen).

The term "gelatin" as used herein refers to a mixture of peptides and proteins produced by partial hydrolysis of collagen extracted from the skin, bones, and connective tissues of animals such as domesticated cattle, chicken, pigs, and fish. During hydrolysis, the natural molecular bonds between individual collagen strands are broken down into a form that rearranges more easily. The term "gelatin" as used herein also encompasses modified gelatins, such a cross-linked gelatins and reduced cross-linked gelatins.

The term "reduced cross-linked gelatins" as used herein refers to a cross-linked gelatin that has been partly hydrolyzed. Partial hydrolysis of the peptide bonds in the cross-linked gelatin can be achieved by e.g. alkaline treatment. Hydrolysis of the cross-linked gelatin results in an increased density of free carboxyl and free amine groups.

The term "gelfoam" as used herein, unless indicated otherwise, refers to a cross-linked gelatin material having a sponge-like structure.

The term "water-insoluble polymer containing reactive nucleophilic groups" refer to a polymer containing reactive nucleophilic groups that has a solubility in demineralized water of 20°C, at pH 7, of less than 5 g/L. In order to determine water solubility of the water-soluble polymer at different pH, the pH of the demineralized water is adjusted using hydrochloric acid
The term "hemostatic sheet" as used herein, unless indicated otherwise, refers to a sheet having the ability to stop bleeding from damaged tissue. The hemostatic sheet of the present invention may achieve hemostasis by turning blood into a gel and/or by forming a seal that closes off the wound site.

The term "water-resistant" as used herein in relation to the fibrous carrier structure means that this structure is not water soluble and does not disintegrate in water to form a colloidal dispersion, at neutral pH conditions (pH 7) and a temperature of 37°C.

The term "interstitial space" as used herein refers to the void ("empty") space within the fibrous carrier structure. The interstitial space within the fibrous carrier structure allows the introduction of hemostatic powder into the structure. Also blood and other bodily fluids can enter the interstitial space, thereby allowing the hemostatic powder to exert its hemostatic effect and/or to provide tissue-adhesiveness to the hemostatic sheet.

The term "tapped density" as used herein refers to the density that is obtained by carefully filling a graduated cylinder (250 mL, internal diameter of 37 mm) with 250 mL of powder followed by mechanically tapping the cylinder until no further volume decrease is observed. The tapped density is calculated as mass divided by the final volume of the powder.

The term "particle" as used herein, unless indicated otherwise, encompasses both particles that consist of a single uniform particle as well as agglomerates of sub-particles. Agglomerates may be prepared by means of granulation techniques known in the art, such as wet granulation.

The term "liquid" as used herein, unless indicated otherwise, means liquid at a temperature of 20°C and a pressure of 1 atmosphere.

The diameter distribution of the sealing powder and of particulate constituents of the sealing powder may suitably be determined by means of laser diffraction using a Malvern Mastersizer 2000 in combination with the Stainless Steel Sample Dispersion Unit. The sample dispersion unit is filled with approx. 120 ml of cyclohexane/diethyl ether (1:1 v/v), which is stabilized for 5 to 10 minutes at a stirring speed of 1800 rpm, followed by a background measurement (blanc measurement). The sample tube is shaken and turned horizontally for 20 times. Next, about 50 mg is dispersed in the sample dispersion unit containing the cyclohexane. After the sample is introduced in the dispersion unit, the sample is stirred for one and a half minute at 1800 rpm to ensure that all particles are properly dispersed, before carrying out the measurement. No ultrasonic treatment is performed on the dispersed particles. Mean particle size is expressed as D [4,3], the volume weighted mean diameter (ΣniDi⁴)/(ΣniDi³).

Besides components (a), (b), (c) and (d) the sealing powder of the present invention may suitably contain one or more other components, such as surfactants, buffering agents and/or polysaccharides. Preferably, the combination of components (a), (b), (c) and (d) constitutes at least 80 wt.%, most preferably at least 90 wt.% of the sealing powder.

The sealing powder preferably has a tapped density in the range of 0.25-0.8 g/ml, more preferably of 0.3-0.6 g/ml.

In accordance with another preferred embodiment, at least 90 wt.% of the sealing powder has a diameter below 500 µm and not more than 10 wt.% of the sealing powder has a diameter below 20 µm. More preferably, at least 90 wt.% of the sealing powder has a diameter below 400 µm and not more than 10 wt.% of the sealing powder has a diameter below 40 µm.

Preferably, at least 50 wt.% of the sealing powder has a diameter in the range of 65-300 µm, more preferably in the range of 80-280, most preferably in the range of 100-200 µm.

The sealing powder preferably has a volume weighted mean diameter (D [4,3]) in the range of 65-300 µm, more preferably of 80-250 µm and most preferably of 125-180 µm.

The water-soluble electrophilic polymer that is contained in the sealing powder of the present invention is preferably selected from electrophilic polyoxazoline, electrophilic polyethylene glycol and combinations thereof.

Preferably, the sealing powder contains 10-50 wt.%, more preferably 20-30 wt.% of the water-soluble electrophilic polymer.

The water-soluble electrophilic polymer preferably has a solubility in demineralized water of 20°C, at a pH in the range of 3 to 7, of at least 100 g/L, more preferably of at least 200 g/L.

The water-soluble electrophilic polymer preferably has a solubility in acetone at 20°C of less than 10 mg/L, more preferably of less than 5 mg/L and most preferably of less than 1 mg/L.

The water-soluble electrophilic polymer preferably has a molecular weight of at least 2 kDa. More preferably, the electrophilic polymer has a molecular weight of 5 to 200 kDa, most preferably of 10 to 100 kDa.

The water-soluble electrophilic polymer and the water-soluble nucleophilic cross-linker are preferably combined in the sealing powder of the present invention with minimum cross-linking reactions between, and minimum degradation of the electrophilic polymer. Accordingly, in a very preferred embodiment of the invention, the water-soluble electrophilic polymer in the sealing powder has a polydispersity index (PDI) of less than 2.0, more preferably of less than 1.8 and most preferably of less than 1.5.

The water-soluble electrophilic polymer preferably contains at least 4 reactive electrophilic groups, more preferably at least 8 reactive electrophilic groups, even more preferably at least 16 reactive electrophilic groups and most preferably at least 32 reactive electrophilic groups.

The water-soluble electrophilic polymer typically carries on average at least 10, more preferably at least 20 reactive electrophilic groups.

According to a particularly preferred embodiment, the electrophilic polymer is electrophilic polyoxazoline.

The electrophilic polyoxazoline is preferably derived from a polyoxazoline whose repeating units are represented by the following formula (I):

(CHR¹)ₘNCOR²

wherein R², and each of R¹ are independently selected from H, optionally substituted C₁₋₂₂ alkyl, optionally substituted cycloalkyl, optionally substituted aralkyl, optionally substituted aryl; and m being 2 or 3.

Preferably, R¹ and R² in formula (I) are selected from H and C₁₋₈ alkyl, even more preferably from H and C₁₋₄ alkyl. R¹ most preferably is H. The integer m in formula (I) is preferably equal to 2.

According to a preferred embodiment, the polyoxazoline is a polymer, even more preferably a homopolymer of 2-alkyl-2-oxazoline, said 2-alkyl-2-oxazoline being selected from 2-methyl-2-oxazoline, 2-ethyl-2-oxazoline, 2-propyl-2-oxazoline, 2-butyl-2-oxazoline and combinations thereof. Preferably, the polyoxazoline is a homopolymer of 2-propyl-2-oxazoline or 2-ethyl-oxazoline. Most preferably, the polyoxazoline is a homopolymer of 2-ethyl-oxazoline.

According to a particularly preferred embodiment, the electrophilic polyoxazoline comprises at least 20 oxazoline units, more preferably at least 30 oxazoline units and most preferably at least 80 oxazoline units.

The electrophilic polyoxazoline preferably comprises on average at least 0.05 reactive electrophilic groups per oxazoline residue. Even more preferably, the electrophilic polyoxazoline comprises on average at least 0.1 reactive electrophilic groups per oxazoline residue. Most preferably, the electrophilic polyoxazoline comprises on average 0.12-0.5 reactive electrophilic groups per oxazoline residue.

Polyoxazoline can carry reactive electrophilic groups in its side chains (pendant reactive electrophilic groups), at its termini, or both. The electrophilic polyoxazoline that is employed in accordance with the present invention advantageously contains one or more pendant reactive electrophilic groups.

Typically, the electrophilic polyoxazoline contains 0.03-0.5 pendant reactive electrophilic groups per monomer, more preferably 0.04-0.35 pendant reactive electrophilic groups per monomer, even more preferably 0.05-0.25 pendant reactive electrophilic groups per monomer.

In accordance with a preferred embodiment, the reactive electrophilic groups of the electrophilic polyoxazoline are selected from carboxylic acid esters, sulfonate esters, phosphonate esters, pentafluorophenyl esters, p-nitrophenyl esters, p-nitrothiophenyl esters, acid halide groups, anhydrides, ketones, aldehydes, isocyanato, thioisocyanato, isocyano, epoxides, activated hydroxyl groups, olefins, glycidyl ethers, carboxyl, succinimidyl esters, sulfo succinimidyl esters, maleimido (maleimidyl), ethenesulfonyl, imido esters, aceto acetate, halo acetal, orthopyridyl disulfide, dihydroxy-phenyl derivatives, vinyl, acrylate, acrylamide, iodoacetamide and combinations thereof. More preferably, the reactive electrophilic groups are selected from carboxylic acid esters, sulfonate esters, phosphonate esters, pentafluorophenyl esters, p-nitrophenyl esters, p-nitrothiophenyl esters, acid halide groups, anhyinidrides, ketones, aldehydes, isocyanato, thioisocyanato, isocyano, epoxides, activated hydroxyl groups, glycidyl ethers, carboxyl, succinimidyl esters, sulfo succinimidyl esters, imido esters, dihydroxy-phenyl derivatives, and combinations thereof. Even more preferably, the reactive electrophilic groups are selected from halo acetals, orthopyridyl disulfide, maleimides, vinyl sulfone, dihydroxyphenyl derivatives, vinyl, acrylate, acrylamide, iodoacetamide, succinimidyl esters and combinations thereof. Most preferably, the reactive electrophilic groups are selected from maleimides, vinyl, acrylate, acrylamide, succinimidyl esters, sulfo succinimidyl esters and combinations thereof.

Examples of succinimidyl esters that may be employed include succinimidyl glutarate, succinimidyl propionate, succinimidyl succinamide, succinimidyl carbonate, disuccinimidyl suberate, bis(sulfosuccinimidyl) suberate, dithiobis(succinimidylpropionate), bis(2-succinimidooxycarbonyloxy) ethyl sulfone, 3,3'-dithiobis(sulfosuccinimidyl-propionate), succinimidyl carbamate, sulfosuccinimidyl(4-iodoacetyl)aminobenzoate, bis(sulfosuccinimidyl) suberate, sulfosuccinimidyl-4-(N-maleimidomethyl)-cyclohexane-1-carboxylate, dithiobis-sulfosuccinimidyl propionate, disulfo-succinimidyl tartarate; bis[2-(sulfo-succinimidyloxycarbonyloxyethylsulfone)], ethylene glycol bis(sulfosuccinimiclylsuccinate), dithiobis-(succinimidyl propionate).

Examples of dihydroxyphenyl derivatives that may be employed include dihydroxyphenylalanine, 3,4-dihydroxyphenylalanine (DOPA), dopamine, 3,4-dihydroxyhydroccinamic acid (DOHA), norepinephrine, epinephrine and catechol.

The water-soluble nucleophilic cross-linker that is employed in the sealing powder of the present invention is preferably selected from nucleophilic polyoxazoline, nucleophilic polyethylene glycol, polyethyleneimine, protein and combinations thereof and combinations thereof. More preferably, the nucleophilic cross-linker is selected from nucleophilic polyoxazoline, nucleophilic polyethylene glycol and combinations thereof. Most preferably, the nucleophilic cross-linker is nucleophilic polyoxazoline.

The water-soluble nucleophilic cross-linker preferably contains at least 3 reactive nucleophilic groups, more preferably at least 4 reactive nucleophilic groups, even more preferably at least 8 reactive nucleophilic groups and most preferably at least 10 reactive nucleophilic groups. Most preferably, these reactive nucleophilic groups are amine groups, most preferably primary amine groups.

In accordance with an embodiment of the invention, the water-soluble nucleophilic cross-linker is nucleophilic polyethylene glycol (PEG). Preferably, the nucleophilic PEG contains at least 3 more preferably at least 5 and most preferably 8 reactive nucleophilic groups

According to a particularly preferred embodiment, the nucleophilic cross-linker is nucleophilic polyoxazoline. Preferably, the nucleophilic polyoxazoline contains at least 3 more preferably at least 5 and most preferably 8 to 20 reactive nucleophilic groups.

Preferably, the nucleophilic polyoxazoline comprises on average at least 0.1 reactive nucleophilic groups per oxazoline residue. Most preferably, the nucleophilic polyoxazoline comprises on average 0.12-0.5 reactive nucleophilic groups per oxazoline residue.

Preferably, the sealing powder contains 1.5-35 wt.%, more preferably 2-20 wt.%, most preferably 3-10 wt.% of the water-soluble nucleophilic cross-linker.

The water-soluble nucleophilic cross-linker preferably has a solubility in demineralized water of 20°C, at pH 7, of at least 100 g/L, more preferably of at least 200 g/L.

The water-soluble nucleophilic cross-linker preferably has a solubility in acetone at 20°C of less than 10 mg/L, more preferably of less than 5 mg/L and most preferably of less than 1 mg/L.

According to a particularly preferred embodiment, the water-soluble nucleophilic cross-linker dissolves relatively slowly in water. It is believed that when the water-soluble electrophilic polymer reacts with the water-soluble nucleophilic cross-linker at a relatively slow rate, the electrophilic polymer can also react with proteins in blood and tissue at the bleeding site. By employing a water-soluble nucleophilic cross-linker that dissolves relatively slowly, dissolved electrophilic polymer has the opportunity to react with proteins in blood and tissue as well as with the (slowly) water-soluble nucleophilic cross-linker, thereby creating a strong homogeneous sealing gel.

Water-soluble nucleophilic cross-linkers having a high molecular weight tend to dissolve relatively slowly in water. Accordingly, in a very preferred embodiment, the water-soluble nucleophilic cross-linker has a molecular weight of at least 3 kDa, more preferably of at least 10 kDa and most preferably of 20 to 3,000 kDa.

The present invention may suitably employ a water-soluble nucleophilic cross-linker that is soluble at acidic pH if the water-soluble electrophilic polymer releases acidic substances when it reacts with nucleophilic groups. This is the case, for instance, when the electrophilic polymer contains N-hydroxysuccinimide groups.

According to a preferred embodiment, the water-soluble nucleophilic cross-linker contains two or more amine groups and the reactive electrophilic groups of the water-soluble electrophilic polymer are selected from carboxylic acid esters, sulfonate esters, phosphonate esters, pentafluorophenyl esters, p-nitrophenyl esters, p-nitrothiophenyl esters, acid halide groups, anhydrides, ketones, aldehydes, isocyanato, thioisocyanato, isocyano, epoxides, activated hydroxyl groups, glycidyl ethers, carboxyl, succinimidyl esters, sulfosuccinimidyl esters, imido esters, dihydroxy-phenyl derivatives, and combinations thereof.

According to another preferred embodiment, the water-soluble nucleophilic cross-linker contains two or more thiol groups and the reactive electrophilic groups of the water-soluble electrophilic polymer are selected from halo acetals, orthopyridyl disulfide, maleimides, vinyl sulfone, dihydroxyphenyl derivatives, vinyl, acrylate, acrylamide, iodoacetamide, succinimidyl esters, sulfosuccinmidyl esters and combinations thereof. More preferably, the reactive electrophilic groups are selected from succinimidyl esters, sulfosuccinimidyl esters, halo acetals, maleimides, or dihydroxyphenyl derivatives and combinations thereof. Most preferably, the reactive electrophilic groups are selected from maleimides or dihydroxyphenyl derivatives and combinations thereof.

The combination of the water-soluble electrophilic polymer and the water-soluble nucleophilic cross-linker preferably constitutes at least 20 wt.%, more preferably 20-60 wt.% and most preferably 25-35 wt.% of the sealing powder.

The ratio between the total number of reactive electrophilic groups provided by the water-soluble electrophilic polymer and the total number of reactive nucleophilic groups provided by the water-soluble nucleophilic cross-linker preferably lies in the range of 1: 0.05 to 1 : 0.4 more preferably in the range of 1 : 0.1 to 1 : 0.3 and most preferably in the range of 1 : 0.15 to 1 : 0.25

The bioresorbable sealing powder of the present invention preferably contains 10-60 wt.%, more preferably 12-40 wt.%, most preferably 15-30 wt.% of the water-absorbing particles containing water-insoluble polymer containing reactive nucleophilic groups.

According to a particularly preferred embodiment, the water-absorbing particles are water-resistant, meaning that these particles are not water soluble and do not disintegrate in water to form a colloidal dispersion, at neutral pH conditions (pH 7) and a temperature of 37°C. Gelfoam particles may suitably be used as water-absorbing particles in accordance with the present invention.

The water-absorbing particles preferably contain at least 50% by weight, more preferably at least 70% by weight of said water-absorbing particles of water-insoluble polymer containing reactive nucleophilic groups selected from amine group, thiol groups and combinations thereof.

In accordance with a particularly preferred embodiment, the water-absorbing particles contain at least 10% by weight, more preferably at least 15% by weight of said water-absorbing particles of water-insoluble polymer containing reactive amine groups.

The water-insoluble polymer containing reactive nucleophilic groups preferably has a water-solubility in demineralized water of 20°C, at pH 7, of less than 3 g/L, more preferably of less than 2 g/L.

The water-insoluble polymer containing reactive nucleophilic groups preferably has a solubility in acetone at 20°C of less than 10 mg/L, more preferably of less than 5 mg/L and most preferably of less than 1 mg/L.

The water-insoluble polymer containing reactive nucleophilic groups that is contained in the water-absorbing particles is preferably selected from proteins, chitosan and combinations thereof. Most preferably, the water-insoluble polymer containing reactive nucleophilic groups is protein.

Chitosan is a biodegradable, nontoxic, complex carbohydrate derivative of chitin (poly-N-acetyl-D-glucosamine), a naturally occurring substance. Chitosan is the deacetylated form of chitin. The chitosan applied in accordance with the present invention preferably has a degree of deacetylation of more than 50%. Chitosan employed in accordance with the present invention preferably has a molecular weight of at least 5 kDa, more preferably of 10-10,000 kDa.

Examples of proteins that may be employed include gelatin, cross linked gelatin, collagen and combinations thereof. More preferably, the water-insoluble polymer containing reactive nucleophilic groups is cross-linked gelatin.

The cross-linked gelatin, preferably has a molecular weight in the range of 30-3,000 kDa, more preferably in the range of 400 to 2,000 kDa, most preferably in the range of 500 to 1,500 kDa.

The average primary amine content of the cross-linked gelatin is preferably in the range of 5 × 10⁻⁴ to 2 × 10⁻² pmol, more preferably 1.0 × 10⁻³ to 1.0 × 10⁻² pmol of primary amine per µg of reduced cross-linked gelatin.

The water-absorbing particles that are employed in the sealing powder of the present invention preferably have a volume weighted mean diameter (D [4,3]) of at least 10 µm, more preferably in the range of 20-250 µm, most preferably in the range of 25-180 µm.

The water-absorbing capacity of the water-absorbing particles is at least 0.2 grams of water per gram of water absorbing particles, more preferably 0.5-3 grams of water per gram of water- absorbing particles and most preferably 0.8-2 grams of water per gram of water absorbing particles. The water-absorbing capacity may suitably be determined gravimetrically.

The water-soluble dispersant that is contained in the sealing powder of the present invention is preferably selected from sucrose, trehalose, lactose, maltitol, mannitol, sorbitol, xylitol, cyclodextrin, maltodextrin, dextran and combinations thereof, more preferably the water-soluble dispersant is selected from sucrose, trehalose, mannitol and combinations thereof.

The water-soluble dispersant preferably has a glass transition temperature above 30°C, more preferably a glass transition temperature of 50-200°C, most preferably a glass transition temperature of 75-95°C.

Preferably, the sealing powder contains 25-70 wt.%, more preferably 30-65 wt.% and most preferably 40-60 wt.% of the water-soluble dispersant.

In a preferred embodiment, the sealing powder contains at least 30 wt.%, more preferably at least 35 wt.% and most preferably at least 40 wt.% of particles that contain both components (a) and (d).

In accordance with another preferred embodiment, the sealing powder contains at least 20 wt.%, more preferably at least 30 wt.% and most preferably at least 40 wt.% of particles comprising both components (b) and (d).

According to a particularly preferred embodiment, the sealing powder contains at least 20 wt.%, more preferably at least 30 wt.% and most preferably at least 40 wt.% of particles comprising both components (b), (c) and (d).

The sealing powder of the present invention may be composed of particles that have the same composition or it can be a mixture of different particles. Examples of mixtures of different particles include:
(a) A mixture of (i) particles only containing components (a) and (d) and (ii) particles only containing components (b), (c) and (d)
(b) A mixture of i) particles only containing components (a) and (d), (ii) particles only containing components (b) and (d) and (iii) particles only containing components (c) and (d).

Here the term "only" is used to indicate that besides the components mentioned, the particles do not contain any of the other components (a) to (d) of the sealing powder.

In case the sealing powder is composed of a mixture of different particles, the powder preferably comprises 30-70 wt.% of particles only containing components (a) and (d) and 30-70 wt.% of particles only containing components (b), (c) and (d). More preferably, the sealing powder comprises 40-60 wt.% of particles only containing components (a) and (d) and 40-60 wt% of particles only containing components (b), (c) and (d).

Preferably, particles only containing components (a) and (d) and the particles only containing components (b), (c) and (d) together constitute at least 60 wt.%, more preferably at least 80 wt.% and most preferably at least 90 wt.% of the sealing powder.

In a particularly preferred embodiment, the present sealing powder contains at least 60 wt.%, more preferably at least 80 wt.% and most preferably at least 90 wt.% of particles containing each of components (a), (b), (c) and (d). Such particles may be prepared, for instance, by means of granulation of mixture of different particles as described above.

According to another particularly preferred embodiment, the sealing powder contains at least 50 wt.%, more preferably at least 70 wt.% and most preferably at least 80 wt.% of particles containing each of the components (a), (b), (c) and (d) in the form of agglomerates of sub-particles A only containing components (a) and (d) and sub-particles B only containing components (b), (c) and (d). Preferably, the agglomerates of sub-particles A and B contain 20-80 wt.% of sub-particles A and 20-80 wt.% of sub-particles B. More preferably, the agglomerates of sub-particles A and B contain 40-60 wt.% of sub-particles A and 40-60 wt.% of sub-particles B.

Preferably, the particles of the sealing powder contain 0.05-5 wt.%, more preferably 0.1-2 wt.% of a surfactant having a melting point of 30°C or higher.

In a preferred embodiment, the surfactant is selected from block copolymer surfactant, polyoxyethylene stearate, sodium dodecyl sulfate and combinations thereof, more preferably a poloxamer, most preferably poloxamer 188 or poloxamer 407.

According to another preferred embodiment, the surfactant is contained in particles that additionally contain components (b) and (d), more preferably in particles that additionally contain components (b), (c) and (d), most preferably in particles that additionally contain components (a), (b), (c) and (d).

Another aspect of the invention relates to a method of treating a wound or reducing bleeding at a hemorrhaging site, comprising topically administering to the wound or hemorrhaging site a sealing powder according to the present invention.

Preferably, in the present method of treatment the powder is topically administered in an amount of 5-250 mg/cm², more preferably in an amount of 20-200 mg/cm², most preferably in an amount of 50-125 mg/cm².

The present method is particularly suited for topical treatment of a) wounds selected from minor abrasions, cuts, scrapes, scratches, burns, sunburns, ulcers, internal venous bleeding, external venous bleeding, and b) surgical interventions selected from gastrointestinal surgery, surgery on parenchymal organs; surgical interventions in the ear, nose and throat area (ENT), cardiovascular surgery, aesthetic surgery, spinal surgery, neurological surgery; lymphatic, biliary, and cerebrospinal (CSF) fistulae, air leakages during thoracic and pulmonary surgery, thoracic surgery, orthopaedic surgery; gynaecological surgical procedures; vascular surgery and emergency surgery, liver resection, and soft tissue injury or surgery.

A further aspect of the invention relates to a method of preparing the bioresorbable sealing powder of the present invention comprising the steps of:
(a) providing particles A comprising the water-soluble electrophilic polymer and the water-soluble dispersant;
(b) providing particles B comprising the water-soluble nucleophilic cross-linker, the water-absorbing particles and the water-soluble dispersant;
(c) combining the particles A and particles B.

In the present method of preparation premature cross-linking reactions between on the one hand the water-soluble electrophilic polymer and on the other hand the water-soluble nucleophilic cross-linker and the water-insoluble polymer containing reactive nucleophilic groups, are effective avoided.

Particles A preferably contain 20-90 wt.% of the water-soluble electrophilic polymer and 10-80 wt.% of the water-soluble dispersant. More preferably, particles A contain 30-70 wt.% of the water-soluble electrophilic polymer and 30-70 wt.% of the water-soluble dispersant. Most preferably, contain 40-60 wt.% of the water-soluble electrophilic polymer and 40-60 wt.% of the water-soluble dispersant.

Together, the water-soluble electrophilic polymer and the water-soluble dispersant preferably constitute at least 60 wt.%, more preferably at least 80 wt.% and most preferably at least 90 wt.% of the particles A.

The particles A are preferably prepared by agglomeration of particles of the water-soluble electrophilic polymer and particles of the water-soluble dispersant. Agglomeration is preferably achieved by means of wet granulation.

Particles B preferably contain 5-20 wt.% of the water-soluble nucleophilic cross-linker, 25-60 wt.% of the water-absorbing particles and 30-70 wt.% of the water-soluble dispersant. More preferably, particles B contain 6-18 wt.% of the water-soluble nucleophilic cross-linker, 30-50 wt.% of the water-absorbing particles and 35-65 wt.% of the water-soluble dispersant. Most preferably, particles B contain 8-15 wt.% of the water-soluble nucleophilic cross-linker, 35-45 wt.% of the water-absorbing particles and 40-60 wt.% of the water-soluble dispersant.

Together, the water-soluble nucleophilic cross-linker, the water-absorbing particles and the water-soluble dispersant preferably constitute at least 60 wt.%, more preferably at least 80 wt.% and most preferably at least 90 wt.% of the particles B.

The particles B are preferably prepared by agglomeration of particles of the water-soluble nucleophilic polymer, particles of the water-insoluble absorbing particles and particles of the water-soluble dispersant. Agglomeration is preferably achieved by means of wet granulation.

The combining of particles A and B is preferably achieved by simple mixing or by granulation (to form agglomerates).

In case the particles A and B are combined by simple mixing, the particles A preferably have an volume weighted mean diameter (D [4,3]) in the range of 25-300 µm, more preferably in the range of 80-250 µm, most preferably in the range of 125-180 µm. In case of simple mixing, the volume weighted mean diameter (D [4,3]) of particles B preferably is in the range of 25-300 µm, more preferably in the range of 80-250 µm, most preferably in the range of 125-180 µm.

Preferably, the particles A and the particles B are combined into agglomerates. According to a particularly preferred embodiment, the particles A and the particles B are combined into the agglomerates by wet granulation. More preferably the particles A and the particles B are combined into agglomerates using a non-aqueous granulation liquid contains at least 60 wt.% of an organic solvent selected from acetone, isopropyl alcohol, ethanol, methanol, diethyl ether, heptane, hexane, pentane, cyclohexane, dichloromethane and mixtures thereof. More preferably, the non-aqueous granulation liquid contains at least 60 wt.%, most preferably at least 85 wt.% of an organic solvent selected from acetone, isopropyl alcohol, ethanol and mixtures thereof. Even more preferably, the non-aqueous granulation liquid contains at least 60 wt.%, most preferably at least 85 wt.% of acetone.

In case the particles A and B are combined by granulation, the particles A preferably have an volume weighted mean diameter (D [4,3]) in the range of 10-200 µm, more preferably in the range of 20-150 µm, most preferably in the range of 25-120 µm. In case of granulation, the volume weighted mean diameter (D [4,3]) of particles B preferably is in the range of 10-200 µm, more preferably in the range of 20-150 µm, most preferably in the range of 25-120 µm.

The non-aqueous granulation liquid preferably contains not more than 1 wt.% water, more preferably not more than 0.1 wt.% water.

The amount of non-aqueous granulation liquid employed in the present method to combine the particles A and B preferably is in the range of 0.5-5% by weight of the combined amount of the particles A and the particles B. More preferably, the amount of non-aqueous granulation liquid employed is in the range of 1-4 %, most preferably 1.5-3 % by weight of the combined amount of the particles A and the particles B.

Yet another aspect of the invention relates to a device for powder application comprising:
- a reservoir containing a bioresorbable sealing powder according to the present invention;
- an elongated hollow tubular structure having a proximal and a distal end, the distal end having a powder outlet, the proximal end being connected to the reservoir,
- a manual air pump attached to the reservoir and arranged to produce an air flow that carries powder from the reservoir via the elongated hollow tubular structure through the powder outlet.

According to a preferred embodiment, the device comprises a porous filter disposed within the reservoir between the air pump and the powder outlet; and wherein the powder is disposed within the reservoir between the filter and the powder outlet, and wherein the pump is in a fluid communication with the powder outlet through the porous filter and through the powder. The filter is preferably impervious to the powder that is contained in the reservoir.

Preferably the manual air pump comprises a bellows.

The sealing powder of the present invention may advantageously be applied in haemostatic sheets to improve the adhesive and haemostatic properties thereof. Accordingly, another aspect of the invention relates to a biocompatible, flexible, hemostatic sheet comprising:
- a cohesive fibrous carrier structure comprising a three-dimensional interconnected interstitial space; and
- the bioresorbable sealing powder according to the present invention;
wherein the sealing powder is distributed within the interstitial space and/or fixated onto the fibrous carrier structure.

The sealing powder may suitably be fixated onto the fibrous carrier structure by an adhesive comprising a bonding agent, preferably a meltable solid bonding agent. Examples of such bonding agents include powders that are polyester, polypropylene, acrylic or polyethylene based.

In accordance with a preferred embodiment, the sealing powder is distributed within the interstitial space of the fibrous carrier.

The cohesive fibrous carrier structure of the haemostatic sheet preferably is water resistant.

According to a particularly preferred embodiment, the haemostatic sheet of the present invention is bioresorbable. Resorption of the carrier structure and the sealing powder typically requires chemical decomposition (e.g. hydrolysis) of the polymers contained therein. Complete resorption of the haemostatic sheet by the human body is typically achieved in 1 to 10 weeks, preferably in 2 to 8 weeks.

The haemostatic sheet of the present invention typically has a non-compressed mean thickness of 0.5-25 mm. More preferably, the non-compressed mean thickness is in the range of 1-10 mm, most preferably in the range of 1.5-5 mm.

The dimensions of the haemostatic sheet preferably are such that the top and bottom of the sheet each have a surface area of at least 2 cm², more preferably of at least 10 cm² and most preferably of 25-50 cm². Typically, the sheet is rectangular in shape and has a length of 25-200 mm, an a width of 25-200 mm.

The haemostatic sheet preferably has a non-compressed density of less than 200 mg/cm³, more preferably of less than 150 mg/cm³ and most preferably of 10-100 mg/cm³.

The haemostatic sheet of the present invention preferably is essentially anhydrous. Typically, the haemostatic sheet has a water content of not more than 5 wt.%, more preferably of not more than 2 wt.% and most preferably of not more than 1 wt.%.

The water absorption capacity of the haemostatic sheet preferably is at least 50%, more preferably lies in the range of 100% to 800%, most preferably in the range of 200% to 500%.

The haemostatic sheet of the present invention is preferably sterile.

The use of a fibrous carrier structure in the haemostatic sheet of the present invention offers the advantage that the sealing powder can be homogeneously distributed throughout this carrier structure without difficulty. Such a homogeneous distribution is much more difficult to achieve in, for instance, foamed carrier structures.

The fibres in the fibrous carrier structure preferably have a mean diameter of 1-500 µm, more preferably of 2-300 µm and most preferably of 5-200 µm. The mean diameter of the fibres can suitably be determined using a microscope.

Typically, at least 50 wt.%, more preferably at least 80 wt.% of the fibres in the fibrous carrier structure have a diameter of 1-300 µm and a length of at least 1 mm.

Preferably, at least 50 wt.%, more preferably at least 80 wt.% of the fibres in the fibrous carrier structure have an aspect ratio (ratio of length to diameter) of at least 1000.

The fibrous carrier structure that is employed in accordance with the present invention preferably is a felt structure, a woven structure or a knitted structure. Most preferably, the fibrous carrier structure is a felt structure. Here the term "felt structure" refers to a structure that is produced by matting and pressing fibres together to form a cohesive material.

The fibrous carrier structure preferably comprises at least 50 wt.%, more preferably at least 80 wt.% and most preferably at least 90 wt.% fibres containing a fiber polymer selected from gelatin, collagen, cellulose, modified cellulose, carboxymethyldextran, poly(lactic-co-glycolic acid) PLGA, sodium hyaluronate/carboxy methylcellulose, polyvinyl alcohol, chitosan and combinations thereof.

According to a particularly preferred embodiment, the fibrous carrier structure comprises at least 50 wt.%, more preferably at least 80 wt.% and most preferably at least 90 wt.% of fibres containing gelatin and/or modified cellulose. The gelatin employed preferably is cross-linked gelatin. The modified cellulose employed preferably is oxidised cellulose and most preferably oxidised regenerated cellulose.

In another preferred embodiment, the fibrous carrier structure comprises at least 50 wt.%, more preferably at least 80 wt.% and most preferably at least 90 wt.% fibres containing at least 80 wt.% of the one or more fibre polymers mentioned above.

Preferred fibrous carrier structures have an open pore structure with a permeability to air of at least 0.1 L/min × cm², more preferably of at least 0.5 L/min × cm². The air permeability is determined in accordance with EN ISO 9237:1995 (Textiles - Determination of the permeability of fabrics to air).

The fibres in the fibrous carrier structure can be produced by means of methods known in the art, such as electrospinning, electro-blown spinning and high speed rotary sprayer spinning. Production of fibrous carrier structure by means of high speed rotary sprayer spinning is described in US 2015/0010612. It is also possible to use commercially available haemostatic fibrous sheets as the fibrous carrier structure.

The sealing powder is preferably present in the haemostatic sheet of the present invention in an amount of 5-90%, more preferably 10-80%, even more preferably 20-75% and most preferably 50-70%, by weight of the fibrous carrier structure.

A further aspect of the invention relates to a kit of parts for preparing a bioresorbable sealing suspension, said kit comprising:
- a first container or compartment comprising a biocompatible liquid; and
- a second container or compartment comprising the bioresorbable sealing powder of the present invention.

A bioresorbable suspension can be prepared using the aforementioned kit by mixing the biocompatible liquid with the sealing powder. The suspension so obtained may be advantageously be applied, for instance, to fill and seal pleural cavities or bleedings.

The biocompatible liquid preferably contains one or more biocompatible liquids selected from polyethylene glycol, propylene glycol, triethyl citrate, polyglycerol, DMSO, glycerol, diacetin, triacetin, N-methyl pyrrolidone (NMP), water and mixtures thereof. The polyethylene glycol used in the suspension preferably has a molecular weight of not more than 550 g/mol, more preferably of not more than 450 g/mol.

The biocompatible liquid of the present kit may suitably contain water. The presence of water in the biocompatible liquid enables the occurrence of cross-linking reactions between the component of the sealing powder before the sealing suspension is applied onto tissue. These initial crosslinking reactions will render the suspension more viscous and sticky which facilitates application of the suspension onto tissue. The water content of the biocompatible liquid preferably is in the range of 1-50 wt.%, more preferably does not exceed 2-30 wt.% and most preferably does not exceed 3-10 wt.%.

The biocompatible liquid may suitably contain buffers, and/or viscosity modifying agents, such as hyaluronic acid, alginate, carboxymethylcellulose and combinations thereof).

Yet another aspect of the present invention relates to a bioresorbable sealing suspension comprising:
- a biocompatible continuous liquid non-aqueous phase; and
- a dispersed phase comprising the bioresorbable sealing powder according to the present invention.

The non-aqueous phase of the sealing suspension preferably contains one or more biocompatible liquids selected from polyethylene glycol, propylene glycol, triethyl citrate, polyglycerol, DMSO, glycerol, diacetin, triacetin, N-methyl pyrrolidone (NMP) and mixtures thereof. The polyethylene glycol used in the suspension preferably has a molecular weight of not more than 550 g/mol, more preferably of not more than 450 g/mol.

The water content of the non-aqueous phase preferably does not exceed 3 wt.%, more preferably does not exceed 1 wt.% and most preferably does not exceed 0.3 wt.%.

Besides the sealing powder, the non-aqueous phase may suitably include other components, such as buffers, and viscosity modifying agents (e.g., hyaluronic acid, alginates and/or carboxymethylcellulose).

The sealing suspension preferably contains 5-75 wt.%, more preferably 10-40 wt.% and most preferably 15-30 wt.% of the sealing powder.

Since over time the particles of sealing powder in the suspension may start to sink or float, the suspension may need to be shaken or stirred before use.

The invention is further illustrated by the following non-limiting examples.

### EXAMPLES

In general: wherever residual moisture (i.e., residual water in dried powder, granulate and/or cohesive fibrous carrier structures) after drying is not explicitly mentioned, levels are below 2.0% w/w.

### Preparation of NHS-POx

NHS-side chain activated poly[2-(ethyl/hydroxy-ethyl-amide-ethyl/NHS-ester-ethyl-ester-ethyl-amide-ethyl)-2-oxazoline] terpolymer containing 20% NHS-ester groups (= EL-POx, 20% NHS) was synthesized as follows:
poly[2-(ethyl/methoxy-carbonyl-ethyl)-2-oxazoline] copolymer (DP = +/-100) was synthesized by means of CROP using 60% 2-ethyl-2-oxazoline (EtOx) and 40% 2-methoxycarbonyl-ethyl-2-oxazoline (MestOx). A statistical copolymer containing 40% 2-methoxycarbonyl-ethyl groups (¹H-NMR) was obtained.

Secondly, the polymer containing 40% 2-methoxycarbonyl-ethyl groups, was reacted with ethanolamine yielding a copolymer with 40% 2-hydroxy-ethyl-amide-ethyl-groups (¹H-NMR). After that, half of the 2-hydroxy-ethyl-amide-ethyl-groups was reacted with succinic anhydride yielding a terpolymer with 60% 2-ethyl groups, 20% 2-hydroxy-ethyl-amide-ethyl-groups and 20% 2-carboxy-ethyl-ester-ethyl-amide-ethyl-groups according to ¹H-NMR. Lastly, the 2-carboxy-ethyl-ester-ethyl-amide-ethyl-groups were activated by N-hydroxysuccinimide (NHS) and diisopropylcarbodiimide (DIC), yielding EL-POx, 20% NHS corresponding to an NHS degree of functionalisation (NHS-DF) of 100%. The NHS-POx contained 20% NHS-ester groups according to ¹H-NMR.

NHS-POx was dissolved between 2-8 °C in water (60 g in 300 mL), cooled at minus 80 °C for half an hour and freeze dried. The freeze dried powder so obtained was dried in a Rotavap at 40 °C until the water content was below 0.8% w/w as determined via Karl Fischer titration. This dry (white) powder was grinded using a ball mill (Retch MM400) until the average particle size was not more than 40 µm (D [4,3]) and vacuum sealed in alu-alu bags.

### Dyeing of NHS-POx powder

31.25 mg of the dye Blue No.1 (CAS 3844-45-9, Spectrum Chem., VWR) was dissolved in 500 mL cold ultrapure water using a high performance dispersing instrument (Ultra-Turrax, IKA). After mixing (5 minutes) 62.5 g NHS-POx was dissolved in the FD&C solution using a high performance dispersing (Ultra-Turrax, IKA). Directly after mixing (5 minutes) the solution was flash freezed and subsequently freeze dried. The freeze dried powder was dried in Rotavap at 40 °C until the residual water content was below 0.8% w/w as determined via Karl Fischer titration. Next, the dried (blue) powder was grinded using a ball mill (Retch MM400) until a blue dyed NHS-POx powder having an average particle size of not more than 40 µm (D [4,3]) and vacuum sealed in alu-alu bags. The NHS-POx co-freeze dried was analyzed using ¹H-NMR spectroscopy. 15 mg of co-freeze dried powder was dissolved in deuterated dimethylsulfoxide (DMSO-*d*₆). The sample was transferred to an NMR tube and a ¹H-NMR spectrum was recorded. From the acquired spectrum, the amount of NHS bound to NHS-POx is calculated: on average the NHS-DF is between 90% to 100%.

### Preparation of NU-POx

Polyoxazoline containing ethyl and amine groups in the alkyl side chain was synthesized by CROP of EtOx and MestOx and subsequent amidation of the methyl ester side chains with ethylene diamine to yield a poly(2-ethyl/aminoethylamidoethyl-2-oxazoline) copolymer (NU-POx).

The NU-POx contained 10% NH₂ according to ¹H-NMR. NU-POx was dissolved between 2-8 °C in water (60 g in 300 mL), cooled at minus 80 °C for half an hour an freeze dried. The freeze dried powder so obtained was dried in a Rotavap at 40 °C until the water content was below 0.8% w/w as determined via Karl Fischer titration. This dry powder was grinded in a knife mill (Retsch GM200 ) until the average particle size was not more than 100 µm (D [4,3]) and vacuum sealed in alu-alu bags.

### Preparation of NHS-POx / sugar mixtures by co-freeze drying (process A1)

Co-freeze dried NHS-POx / sugar powders were prepared as follows:
15 g of sugar was dissolved in 200 mL cold ultrapure water using a high performance dispersing instrument (Ultra-Turrax, IKA). Subsequently, after mixing (3 minutes) 15 g blue dyed NHS-POx was dissolved in the sugar mixture using a high performance dispersing instrument (Ultra-Turrax, IKA). Directly after mixing (3 minutes) the solution was frozen in liquid nitrogen and freeze dried.

The freeze dried powder was dried in a Rotavap at 40 °C until the residual water content was below 0.8% w/w as determined via Karl Fischer titration. This dry powder was grinded in a knife mill until the particle size was not more than 63 µm and vacuum sealed in alu-alu bags. The NHS-POx/sugar co-freeze dried (1:1 w/w) was analyzed using ¹H-NMR spectroscopy: 15 mg of co-freeze dried powder was dissolved in deuterated dimethylsulfoxide (DMSO-*d*₆). The sample was transferred to an NMR tube and a ¹H-NMR spectrum was recorded. From the acquired spectrum, the NHS-DF was calculated and, on average, between 85% and 100%.

### Preparation of NHS-POx / sugar granulate (process A2)

NHS-POx / sugar granulate was prepared as follows:
23 g of co-freeze dried NHS-POx / sugar as per process A1 was added to a mortar. Subsequently, 10 mL of acetone: water (95:5 v/v) was added slowly in portions of 1 mL, during mixing. The granulate was dried under reduced pressure for 1 hour in a Rotavap at 40 °C and milled for 10 seconds in a knife mill. The milled granulate was dried again under reduced pressure until the acetone content was less than 0.2% and the water content less than 0.8% as determined via ¹H-NMR and Karl Fischer titration.

The dried granulate was milled again until the particle size was in the desired range (see below) and vacuum sealed in alu-alu bags.

The NHS-POx/sugar granulate (1:1 w/w) was analyzed using 1H-NMR spectroscopy: 25 mg of granulate was dissolved in deuterated dimethylsulfoxide (DMSO-d6) containing maleic acid (3 mg/mL) as an internal standard (1.0 mL), transferred to an NMR tube and a 1H-NMR spectrum was recorded. From the acquired spectrum, the NHS-DF was calculated and, on average, between 85% and 100%.

### Preparation of NU-POx / gelfoam / sugar granulate (process B1)

0.2 g NU-POx, 0.8 g gelfoam (unless indicated otherwise: GELITA-SPON powder, ex Gelita Medical AG, Germany) and 0.8 g sugar were weighed and put in a mortar. The excipients were granulated with 50% w/w ultrapure water. The wet granulate was dried in an oven at 70 °C until the water content was less than 0.5% as determined via Karl Fischer titration. The dried granulate was milled until it had the desired particle size range (see below). Reactivity of the granulate may be controlled by adjusting pH to 9.5 with NaOH or to 9.2 using a borate buffer, prior to drying

### Preparation of NU-POx / gelfoam / sugar / surfactant granulate (process B2)

NU-POx / gelfoam / sugar granulate was prepared as described in process B1 with the addition of surfactant dissolved in ultrapure water used for granulation leading to 0.10 - 0.50% w/w surfactant in the final granulate containing NHS-POx / sugar and NU-POx / Gelfoam / sugar.

### Preparation of NHS-POx sugar / NU-POx / gelfoam / sugar / surfactant powder mixture (process C1)

A powder mixture was prepared as follows:
NHS-POx sugar powder (process A2, particle size as desired) and NU-POx / gelfoam / sugar / surfactant granulate (process B1 or B2, particle size as desired) were added in a weight ratio of 1:1 into a vial and mixed by shaking and vacuum sealed in alu-alu bags.

### Preparation of NHS-POx sugar/ NU-POx / gelfoam / sugar/ surfactant powder mixture (process C2)

A powder mixture was prepared as follows:
NHS-POx sugar powder (process A1, particle size <63 µm) and NU-POx / gelfoam / sugar / surfactant granulate (process B1 or B2, particle size as desired) were added into a mortar in a weight ratio of 1:1 and dry mixed and vacuum sealed in alu-alu bags.

### Preparation of NHS-POx sugar / NU-POx / gelfoam / sugar / surfactant granulate (process C3).

Granulate was prepared as follows: NHS-POx sugar granulate (process A2, particle size <63 µm) and NU-POx / gelfoam / sugar / surfactant granulate (process B2, particle size <63 µm) were introduced into a mortar in a weight ratio of 1:1. Subsequently, in total 1 mL/g of acetone was added in portions of 1 mL during grinding and mixing.

The granulate so obtained was dried under reduced pressure until the acetone content was less than 0.2% as determined via ¹H-NMR. The dried granulate was grinded in a mortar until the particle size was between 125 - 180 µm and vacuum sealed in alu-alu bags.

The NHS-POX/sugar granulate (1:1) was analyzed using 1H-NMR spectroscopy. 25 mg of granulate was dissolved in deuterated dimethylsulfoxide (DMSO-d6) containing maleic acid (3 mg/mL) as an internal standard (1.0 mL), transferred to an NMR tube and a 1H-NMR spectrum was recorded. From the acquired spectrum, the NHS-DF was calculated and, on average, between 85% and 100%.

### Milling and sieving

Milling was performed with either mortar and pestle or a knife mill (Retsch GM 200). Sieving was performed to obtain fractions with desired particle sizes and particle size distributions. For this a Retsch AS 200 sieving tower was used with sieves in the following sizes: 45 µm, 63 µm, 90 µm, 125 µm, 180 µm, 250 µm and 500 µm. For obtaining the desired particle size, powders were milled and sieved; by sampling the desired sieving fractions, suitable powder particle size ranges were obtained.

### Application of powders on punch bleedings, abrasion bleedings and lung lesions

Punch bleedings (by vial): a quantity of powder contained in a closed vial with a diameter of 15 mm. The powder was applied by pouring from the vial directly on the punch bleeding.

Once an adequate coverage of the bleeding was achieved, pressure with 0.9% NaCl wet gauze was applied for 1 minute and the wet gauze was carefully removed.

Abrasions (by bellows): a quantity of powder was applied via a bellow applicator with a 80 mm long applicator tube. The powder was applied by blowing out the powder in puffs directly on the lesion (abrasion) and hemostatic efficacy was assessed after 1 minute. In case no (total) hemostatic coverage of the area of abrasion was achieved, additional pressure with 0.9% NaCl wet gauze was applied for 1 minute and the wet gauze was carefully removed.

Lung lesion: powder was manually applied to the lung surface in a 5x5cm square surrounding the standardized defect. Once a homogenous coverage was achieved, pressure with 0.9% NaCl wet gauze was applied for 2 minutes, while adjusting the hand position after 1 minute to prevent irregularities in the final hydrogel and the wet gauze was carefully removed.

### Experiments on hemostasis and sealing

Standardized *ex-vivo* and *in-vivo* porcine bleeding models were used to assess hemostatic efficacy. All models use heparin to increase clotting time of blood to about 2 to 3 times activated coagulation time (ACT).

*Ex-vivo* model: live *ex-vivo* pig model with a fresh liver, perfused with heparinized fresh blood from the slaughterhouse to mimic real *in-vivo* situations a closely as possible. Livers are mounted onto a perfusion machine by which oxygenation, pH of blood, temperature and blood pressure are kept within *vivo* boundaries. Two livers and 10 litres of heparinized blood (5000 units/L) are collected at the slaughterhouse. Livers are transported on ice; blood at ambient temperature. Within two hours after collection, livers are inspected for lesions which are closed with gloves and cyanoacrylate glue.
- Perfusion parameters: flow 600 ml/min; pressure 10-12 mmHg; temperature 37 °C (+/- 1 °C); carbogen 0.25 liters a minute;
- With a biopsy punch a circular bleeding wound (8 mm diameter) is created on the liver surface, with a rubber onlay so that the depth of the punched bleeding is always 3 mm, or alternatively abraded lesions (3x3 cm) are created (depth appr. 1 mm) using sanding paper;
- After the liver is perfused properly (color and temperature checked) samples are tested according to the following procedure: camera on; site number on camera; biopsy punch 8 mm; cut away biopt; remove blood from bleeding with gauze (2x); collect blood for 30 sec in pre-weight gauze; score bleeding;
- apply hemostatic powder on bleeding site (punch or abrasions) and, in case of a punch bleeding use wet gauze (saline) to distribute powder and press for one minute;
- observe and score sealing and hemostasis.

In-vivo model: standardized combined penetrating spleen rupture is inflicted in anesthetized swine (Domestic Pig, Male, Body Weight Range: 40 kg - 100 kg Adult). A midline laparotomy is performed to access the spleen and other organs. Using a scalpel, n=3 subcapsular standardized lesions (10 mm × 10 mm) are made. The hemostatic powders were applied with gentle pressure by a pre-wetted gauze (saline) and held for 1 min after which sealing and hemostasis was scored.

### Sealing experiments

A standardized *ex-vivo* ventilated model of the porcine lung was used to asses sealing capabilities in the absence of blood, being aerostatic efficacy, more specifically.

Ex-vivo model: freshly harvested porcine cardio-pulmonary specimens were ordered from the slaughterhouse and transported to the research facility on ice. After removal of all excess tissues, tying off of the main pulmonary artery and suturing the left atrial remnants, the caudal lobes were selectively intubated and ventilated after alveolar recruitment using manual inflation and manual recruitment maneuvers.

In the experimental setup, lungs floated on 0.9% NaCl (37°C) and were filmed from below for visual leak assessment. A measurement protocol under increasing plateau ventilatory pressures (Pplat) was performed during each measurement. The mechanical ventilator settings were pressure control ventilation, respiratory rate 12/min, inspiratory to expiratory ratio 1:2, positive end-expiratory pressure (PEEP) 5 cm H₂O, pressure above PEEP 5 cm H₂O. Every 90 seconds, pressure above PEEP was increased with 5cm H₂O until a Pplat of 40 cm H₂O.
- First, a baseline measurement was performed with this measurement protocol, in order to do a compliance measurement.
- Then, with the lung inflated at 10 cm H₂O PEEP, a standardized 25x25mm superficial pleural defect was created on the dorsal aspect of the caudal lobe by cutting the pleural edges using a sanding wheel attachment on a Dremel at a 45° angle and then carefully peeling away the center pleura using tweezers and scissors. A baseline leak measurement with the same protocol was then performed.
- Sealing powder was then manually applied to the lung surface in a 5x5 cm square surrounding the standardized defect with the lung inflated at 0-5cm H₂O PEEP (if air was leaking through the dry powder, the PEEP was turned down until this resolved). Once a homogenous coverage was achieved, pressure with 0.9% NaCl wet gauze was applied for 2 minutes, while adjusting the hand position after 1 minute to prevent irregularities in the final hydrogel. After the gauze had been carefully removed, hydrogel is allowed to set for another 5 minutes in the measurement setup (0.9% NaCl, 37°C)
- Quantitative leakage (Pplat)was measured and mode of failure was visually assessed based on Macchiarini et al (Macchiarini P, Wain J, Almy S, Dartevelle P. Experimental and clinical evaluation of a new synthetic, absorbable sealant to reduce air leaks in thoracic operations. J Thorac Cardiovasc Surg. 1999;117(4):751-8).

Sealing scoring system for powders on bleedings (based on assessment of adhesion and cohesion 1 minute after application):
+++ Very strong seal (seal breaks only when more than 80 wt.% of the powder is removed by scraping)
++ Strong seal (seal breaks when part of the powder is removed by scraping)
+ Good seal (seal breaks when powder is mechanically manipulated)
+/- moderate seal (seal breaks when surrounding tissue is manipulated)
- no sealing is achieved

Hemostasis scoring system for powders on bleedings at 1 minute after application:
+++ Very Strong hemostasis (powder is only filled with blood at the powder-lesion interface; bleeding is stopped)
++ Strong hemostasis (powder is partly filled with blood. A layer of powder, free of blood is present on top; bleeding is stopped)
+ Good hemostasis (powder is filled with blood completely; bleeding is stopped)
+/- moderate hemostasis (powder is filled with blood completely and some blood comes through)
- No hemostasis is achieved (bleeding is not stopped)

Wetting scores for powders on bleedings are determined after a gauze soaked with saline (0.9% NaCl) has been applied for 1 minute before being removed (the wetting is scored 4 minutes after removal of the gauze by determining the depth of penetration into the powder layer):
- Saline is poorly absorbed by the powder layer and only the surface of the powder layer is wet and droplets of saline easily roll off the powder surface
+/- Saline has partially penetrated the powder layer
+ Saline has fully penetrated the powder layer

### Comparative Example A

EL-POx was dry mixed by co-grinding (with mortar and pestle) with either gelfoam alone (EL-POx : gelfoam = 1 : 0.8 w/w) or with gelfoam and NU-POx (EL-POx : gelfoam : Nu-POx = 1 : 0.8 : 0.2 w/w). The tap density of both powders was less than 0.2 g/mL.

The sealing and hemostatic properties of these two powders and of two commercially available hemostatic powders were tested in the ex *vivo* pig liver system (heparinized). The powders were applied in an amount of 1 gram.

The test results are summarized in Table 1.

**Table 1**

| **Powder** | **Sealing** | **Hemostasis** | **Wetting** |
|---|---|---|---|
| NHS-POx + gelfoam ¹ | - | +/- | +/- |
| NHS-POx + gelfoam ¹ + NU-POx | - | +/- | +/- |
| Starch ² | - | - | ++ |
| Gelfoam ¹ | - | - | +/- |

| | | | |
|---|---|---|---|
| ¹ GELlTA-SPON^{®} powder, Gelita Medical AG, Germany ² Arista AH, Bard, USA | | | |

### Example 2

A powder mix (Powder mix 1) was prepared by means of process C2 using a powder obtained by process A1 (EL-POX : sugar = 1:1 w/w) and a powder obtained by process B1. In both powders trehalose was used as the sugar component. A powder mix of the same composition (Powder mix 2) was prepared by means of process C1 using a powder obtained by process A2 (EL-POX : sugar =1:1 w/w, granulated using acetone/water (95:5) as granulation liquid) and a powder obtained by process B1.

The sealing and hemostatic properties of these two sealing powders were tested in the ex *vivo* pig liver system (heparinized). The powders were applied in an amount of 0.5 gram.

The test results are summarized in Table 2.

**Table 2**

| **Powder** | **Particle size (µm)** | **Sealing** | **Hemostasis** | **Wetting** |
|---|---|---|---|---|
| Powder mix 1 | <63 | +/- | +/- | + |
| Powder mix 2 | 63-125 | + | + | + |

### Example 3

Three different powder mixes were prepared by means of process C1 using a powder obtained by process A2 (EL-POX : sugar = 1:1 w/w) and three different powders obtained by process B1. In all powders trehalose was used as the sugar component.

The three powder mixes differed only in that the powders obtained by process B1 contained different amounts of NU-POx:

| | **Weight ratio of NU:POx : gelfoam : sugar in powder obtained by process B1** |
|---|---|
| Powder mix 1 | 2:8:8 |
| Powder mix 2 | 1:8:8 |
| Powder mix 3 | 3:8:8 |

The sealing and hemostatic properties of these two sealing powders were tested in the ex *vivo* pig liver system (heparinized). The powders were applied in an amount of 0.5 gram.

The test results are summarized in Table 3.

**Table 3**

| **Powder** | **Particle size (µm)** | **Sealing** | **Hemostasis** | **Wetting** |
|---|---|---|---|---|
| Powder mix 1 | 63-125 | + | + | + |
| Powder mix 2 | 63-125 | - | +/- | + |
| Powder mix 3 | 63-125 | + | + | + |

### Example 4

Two different powders were prepared by mixing 1 part by weight of NHS-POx powder (without sugar) with either 2 parts by weight of a powder obtained by process B1 and containing trehalose or 1 part by weight of a powder obtained by process B1 that does not contain any sugar.

The compositions of the powders obtained by process B1 were as follows:

| | **Weight ratio of NU:POx : gelfoam : sugar in powder obtained by process B1** |
|---|---|
| Powder mix 1 | 2:8:8 |
| Powder mix 2 | 2:8:0 |

The sealing and hemostatic properties of these two sealing powders were tested in the ex *vivo* pig liver system (heparinized). The powders were applied in an amount of 0.5 gram using a bellows applicator (Surgicel^{®}).

The test results are summarized in Table 4.

**Table 4**

| **Powder** | **Particle size (µm)** | **Sealing** | **Hemostasis** | **Wetting** |
|---|---|---|---|---|
| Powder mix 1 | 125-180 | +/- | +/- | +/- |
| Powder mix 2 | Appr. 500 | - | +/- | - |

Powder mix 1 could easily be applied by means of the bellows applicator. However, powder mix 2 was difficult to apply because the powder particles were very fluffy. To allow comparability of the sealing, hemostasis and wetting properties, in this experiment, Powder mix 2 was applied with a tube.

### Example 5

Five different powder mixes were prepared by means of process C1 using a powder obtained by process A2 (EL-POX : sugar =1:1 w/w) and five different powders obtained by process B1. In all powders trehalose was used as the sugar component.

The five different powder mixes differed only in that the powders obtained by process B1 contained different amounts of trehalose:

| | **Weight ratio of NU:POx : gelfoam : sugar in powder obtained by process B1** |
|---|---|
| Powder mix 1 | 2:8:8 |
| Powder mix 2 | 2:8:2 |
| Powder mix 3 | 2:8:4 |
| Powder mix 4 | 2:8:12 |
| Powder mix 5 | 2:8:16 |

The sealing and hemostatic properties of these two sealing powders were tested in the ex *vivo* pig liver system (heparinized). The powders were applied in an amount of 0.5 gram using a bellows applicator.

The test results are summarized in Table 5.

**Table 5**

| **Powder** | **Particle size (µm)** | **Sealing** | **Hemostasis** | **Wetting** |
|---|---|---|---|---|
| Powder mix 1 | 125-180 | + | + | + |
| Powder mix 2 | 125-180 | - | +/- | - |
| Powder mix 3 | 125-180 | +/- | + | -/+ |
| Powder mix 4 | 125-180 | +/- | + | + |
| Powder mix 5 | 125-180 | +/- | +/- | + |

Powder mixes 1, 3, 4 and 5 could easily be applied with a bellows applicator. Powder mix 2 was less easy to apply because the particles were quite fluffy.

### Example 6

Three different powder mixes were prepared by means of process C1 using a powder obtained by process A2 (EL-POX : sugar = 1:1 w/w) and three different powders obtained by process B1. The three different powder obtained by process B1 only differed in the type of sugar that was used. In all cases NU-POx, gelfoam and sugar were present in the powder from process B in a weight ratio of 2:8:8.

| | Type of sugar used |
|---|---|
| Powder mix 1 | Trehalose |
| Powder mix 2 | Mannitol |
| Powder mix 3 | Dextran 40 |

The sealing and hemostatic properties of these two sealing powders were tested in the ex *vivo* pig liver system (heparinized). The powders were applied in an amount of 0.5 gram.

The test results are summarized in Table 6.

**Table 6**

| **Powder** | **Particle size (µm)** | **Sealing** | **Hemostasis** | **Wetting** |
|---|---|---|---|---|
| Powder mix 1 | 125-180 | + | + | + |
| Powder mix 2 | 125-180 | + | + | + |
| Powder mix 3 | 125-180 | +/- | + | + |

### Example 7

Four different powder mixes were prepared by means of process C1 using a powder obtained by process A2 (EL-POX : sugar = 1:1 w/w), one powder obtained by process B1 and three different powders obtained by process B2. The three different powder obtained by process B2 differed in the amount of surfactant (Pluronic F-127) that was used. In all powders trehalose was applied as the sugar component.

| | Wt.% Pluronic F-127 |
|---|---|
| Powder mix 1 | 0 |
| Powder mix 2 | 0.1 |
| Powder mix 3 | 1.0 |
| Powder mix 4 | 3.0 |

The sealing and hemostatic properties of these two sealing powders were tested in the ex *vivo* pig liver system (heparinized). The powders were applied in an amount of 0.5 gram.

The test results are summarized in Table 7.

**Table 7**

| **Powder** | **Particle size (µm)** | **Sealing** | **Hemostasis** | **Wetting** |
|---|---|---|---|---|
| Powder mix 1 | 63-125 | + | + | + |
| Powder mix 2 | 63-125 | ++ | ++ | ++ |
| Powder mix 3 | 63-125 | ++ | ++ | ++ |
| Powder mix 4 | 63-125 | ++ | ++ | ++ |

### Example 8

Example 7 was repeated except that this time sodium dodecyl sulfate (SDS) was used as surfactant.

| | Wt.% SDS |
|---|---|
| Powder mix 1 | 0 |
| Powder mix 2 | 0.1 |
| Powder mix 3 | 1.0 |
| Powder mix 4 | 3.0 |

The sealing and hemostatic properties of these two sealing powders were tested in the ex *vivo* pig liver system (heparinized). The powders were applied in an amount of 0.5 gram.

The test results are summarized in Table 8.

**Table 8**

| **Powder** | **Particle size (µm)** | **Sealing** | **Hemostasis** | **Wetting** |
|---|---|---|---|---|
| Powder mix 1 | 63-125 | + | + | + |
| Powder mix 2 | 63-125 | ++ | ++ | ++ |
| Powder mix 3 | 63-125 | ++ | ++ | ++ |
| Powder mix 4 | 63-125 | ++ | ++ | ++ |

### Example 9

Example 7 was repeated except that this time Poloxamer P-188 was used as surfactant.

| | Wt.% Poloxamer P-188 |
|---|---|
| Powder mix 1 | 0 |
| Powder mix 2 | 0.1 |
| Powder mix 3 | 1.0 |
| Powder mix 4 | 3.0 |

The sealing and hemostatic properties of these two sealing powders were tested in the ex *vivo* pig liver system (heparinized). The powders were applied in an amount of 0.5 gram.

The test results are summarized in Table 9.

**Table 9**

| **Powder** | **Particle size (µm)** | **Sealing** | **Hemostasis** | **Wetting** |
|---|---|---|---|---|
| Powder mix 1 | 63-125 | + | + | + |
| Powder mix 2 | 63-125 | ++ | ++ | ++ |
| Powder mix 3 | 63-125 | ++ | ++ | ++ |
| Powder mix 4 | 63-125 | ++ | ++ | ++ |

### Example 10

Three different powder mixes were prepared by means of process C1 using one powder obtained by process A2 (EL-POX : sugar =1:1 w/w) and three different powders obtained by process B1. The three powders obtained by process B2 differed in that they contained different types of gelfoam.

| | **Gelfoam** |
|---|---|
| Powder mix 1 | GELITA-SPON powder ¹ |
| Powder mix 2 | Cutanplast powder ² |
| Powder mix 3 | Surgispon powder ³ |

| | |
|---|---|
| ¹ Gelita Medical AG, Germany ² Mascia Brunelli S.p.a., Italy ³ Aegis Lifesciences PVT Ltd, India | |

The sealing and hemostatic properties of these sealing powders were tested in the ex *vivo* pig liver system (heparinized). The powders were applied in an amount of 1.0 gram.

The test results are summarized in Table 10.

**Table 10**

| **Powder** | **Particle size (µm)** | **Sealing** | **Hemostasis** | **Wetting** |
|---|---|---|---|---|
| Powder mix 1 | 63-125 | + | + | + |
| Powder mix 2 | 63-125 | + | + | + |
| Powder mix 3 | 63-125 | + | + | + |

### Example 11

Different powder mixes were prepared by means of process C1 using a powder obtained by process A2 (EL-POX : sugar =1:1 w/w) and a powder obtained by process B1. In all powders trehalose was applied as the sugar component. The particle size of the powders was varied as shown in Table 11.

The sealing and hemostatic properties of these sealing powders were tested in the ex *vivo* pig liver system (heparinized). The powders were applied in an amount of 0.5 gram.

The test results are summarized in Table 11.

**Table 11**

| **Powder** | **Particle size (µm)** | **Sealing** | **Hemostasis** | **Wetting** |
|---|---|---|---|---|
| Powder mix 1 | 63 - 125 | + | + | + |
| Powder mix 2 | 125 - 250 | + | + | + |
| Powder mix 3 | 250 - 500 | - | - | + |
| Powder mix 4 | 63 - 90 | + | + | + |
| Powder mix 5 | 90 - 125 | ++ | ++ | + |
| Powder mix 6 | 125 - 180 | ++ | ++ | + |
| Powder mix 7 | 180 - 250 | + | +- | + |

### Example 12

Different powder mixes were prepared by means of powder mixing process C1 or C2, or by granulation process C3, using a powder obtained by process A1 or A2 (EL-POX : sugar = 1:1 w/w), and a powder obtained by process B2.

| | Particles containing NHS-POx | Particles containing NU-POx | Combining of powders |
|---|---|---|---|
| Powder mix 1 | Process A1 | Process B2 | Process C2 |
| Powder mix 2 | Process A2 | Process B2 | Process C1 |
| Powder mix 3 | Process A2 | Process B2 | Process C3 |

In all powders trehalose was applied as the sugar component.

The sealing and hemostatic properties of these two sealing powders were tested in the ex *vivo* pig liver system (heparinized). The powders were applied in an amount of 0.5 gram.

The test results are summarized in Table 12

**Table 12**

| **Powder** | **Particle size (µm)** | **Sealing** | **Hemostasis** | **Wetting** |
|---|---|---|---|---|
| Powder mix 1 | <63 | + | +/- | + |
| Powder mix 2 | 125-180 | ++ | ++ | + |
| Granulate 3 | 125-180 | +++ | +++ | + |

### Example 13

*In vivo* tests were conducted on porcine spleen (heparinized) using powder mixes (0.5 gram) that were prepared by means of process C1 using a powder obtained by process A2 (EL-POX : sugar = 1:1 w/w) and a powder obtained by process B1. In all powders trehalose was applied as the sugar component. The powders only differed in particle size. The test results are summarized in Table 13.

**Table 13**

| **Powder** | **Particle size (µm)** | **Sealing** | **Hemostasis** | **Wetting** |
|---|---|---|---|---|
| Powder mix 1 | 90 - 125 | + | + | + |
| Powder mix 2 | 125-180 | ++ | + | + |

### Example 14

The performance of the powders mixes of Example 12 were also evaluated in the *in* vivo spleen test (heparinized) using 0.5 gram of powder mix. The results are shown in Table 14.

**Table 14**

| **Powder** | **Particle size (µm)** | **Sealing** | **Hemostasis** | **Wetting** |
|---|---|---|---|---|
| Powder mix 1 | 90 - 125 | + | + | + |
| Powder mix 2 | 125-180 | ++ | + | + |

### Example 15

*Ex vivo* tests were conducted on porcine livers (heparinized) having abraded lesions (3x3 cm). The sealing powders used (1 gram) were prepared by means of dry mixing process C1 (Powder mix 1) or granulation process C3 (Granulate 2). In both cases a powder obtained by process A2 and a powder obtained by process B2 were used. The powders were administered using a bellows applicator. In all powders trehalose was applied as the sugar component.

The test results are summarized in Table 15.

**Table 15**

| **Powder** | **Particle size (µm)** | **Sealing** | **Hemostasis** | **Wetting** |
|---|---|---|---|---|
| Powder mix 1 | 125-180 | ++ | ++ | + |
| Granulate 2 | 125-180 | +++ | ++ | + |

### Example 16

The sealing properties of sealing powders in the absence of blood was assessed in the ex *vivo* ventilated lung model described herein before. After application of the sealing powder, the pressure at which leaking started was recorded.

The sealing powders tested were obtained by either dry mixing (process C1) or granulating (process C3) a NHS-POx containing powder and a NU-POx containing powder. The NHS-POx containing powder either consisted of NHS-POx or it was a granulate of NHS-POx and trehalose that had been obtained by process A2. The NU-POx containing powder was granulate that further contained gelfoam and trehalose and that was obtained by process B1 or B2.

The results of the tests are summarized in Table 16.

**Table 16**

| **Powder** | **Particle size (µm)** | **Weight ratio NHS-POx : trehalose** | **Wt.% surfactant ¹** | **Grams applied** | **Leaking pressure (Pplat)** |
|---|---|---|---|---|---|
| Powder mix 1 | 63-125 | 1:0 | 0 | 3.0 | 3 |
| Powder mix 2 | 63-125 | 1:0 | 0 | 3.0 | 8 |
| Powder mix 3 | 63-125 | 1:1 | 0 | 3.0 | 20 |
| Powder mix 4 | 63-125 | 1:1 | 0.1 | 3.0 | 25 |
| Powder mix 5 | 63-125 | 1:1 | 0.5 | 3.0 | 25 |
| Granulate 6 | 125-180 | 1:1 | 0.5 | 1.5 | 42 |

| | | | | | |
|---|---|---|---|---|---|
| ¹ Pluronic F-177 | | | | | |

### Example 17

Granulates were prepared by means of granulation process C3, using a powder obtained by process A2 (EL-POX : trehalose = 1:1 w/w), and a powder obtained by process B2 (particles containing NU-POx), except that in each case the Gelfoam in the latter powder was replaced by another water-insoluble polymer containing reactive nucleophilic groups as shown below:

| | **water-insoluble polymer containing reactive nucleophilic groups** |
|---|---|
| Granulate 1 | Chitosan ¹ |
| Granulate 2 | Reduced crosslinked gelatin ² |

| | |
|---|---|
| 1 ex Sigma Aldrich, MW 100,000-300,000, deacetylation grade of 85% 2 Prepared by the procedure described in WO 2021/009014 (page 29, lines 3-14). | |

The sealing and hemostatic properties of these two sealing granulates were tested in the ex *vivo* pig liver system (heparinized). The granulates were applied in an amount of 0.5 gram. Tests were performed in duplicate. The test results are summarized in Table 17.

**Table 17**

| | **Sealing** | **Hemostasis** | **Wetting** |
|---|---|---|---|
| Granulate 1 | ++ | ++ | + |
| Granulate 2 | ++ | ++ | + |

### Comparative Example B

A granulate was prepared by means of granulation process C3, using a powder obtained by process A2 (EL-POX : trehalose =1:1 w/w), and a powder obtained by process B2 (particles containing NU-POx), except that in this case the Gelfoam was replaced by oxidised resorbable cellulose (GeltaCel^{®}, ex Gelita Medical GmbH).

The sealing and hemostatic properties of the granulate was tested in the ex *vivo* pig liver system (heparinized). The granulate was applied in an amount of 0.5 gram. Tests were performed in duplicate. The test results are summarized in Table 18

**Table 18**

| | **Sealing** | **Hemostasis** | **Wetting** |
|---|---|---|---|
| Granulate | - | - | - |

### Example 18

A granulate was prepared by means of granulation process C3, using a powder obtained by process A2 (EL-POX : sugar =1:1 w/w), and a powder obtained by process B2 (particles containing NU-POx), except that in this case the trehalose in the first powder was replaced by mannitol.

The sealing and hemostatic properties of the granulate was tested in the ex *vivo* pig liver system (heparinized). The granulate was applied in an amount of 0.5 gram. Tests were performed in duplicate. The test results are summarized in Table 19

**Table 19**

| | **Sealing** | **Hemostasis** | **Wetting** |
|---|---|---|---|
| Granulate | ++ | ++ | + |

### Example 19

A granulate was prepared by means of granulation process C3, using a powder obtained by process A2 (EL-POX : sugar =1:1 w/w), and a powder obtained by process B2, except that in the latter powder instead of NU-POx an 8-arm polyethylene glycol carrying 8 amine groups (8-Arm PEG-NH@ ex Creative PEGWorks) was used.

The sealing and hemostatic properties of the granulate was tested in the ex *vivo* pig liver system (heparinized). The granulate was applied in an amount of 0.5 gram. Tests were performed in duplicate. The test results are summarized in Table 20

**Table 20**

| | **Sealing** | **Hemostasis** | **Wetting** |
|---|---|---|---|
| Granulate | + | + | + |

## Claims

1. A bioresorbable sealing powder comprising
(a) at least 5 wt.% of water-soluble electrophilic polymer carrying at least 3 reactive electrophilic groups that are capable of reacting with amine groups under the formation of a covalent bond;
(b) 1-50 wt.% of water-soluble nucleophilic cross-linker carrying at least 2 reactive nucleophilic groups that, in the presence of water, are capable of reacting with the reactive electrophilic groups of the electrophilic polymer under the formation of a covalent bond between the electrophilic polymer and the nucleophilic cross-linker;
(c) 1-60 wt.% of water-absorbing particles containing at least 50 wt.%, calculated by weight of the water-absorbing particles, of water-insoluble polymer containing reactive nucleophilic groups selected from amine groups, thiol groups and combinations thereof;
(d) 10-75 wt.% of water-soluble dispersant that is solid at 20°C, said water-soluble dispersant being selected from monosaccharides, disaccharides, oligosaccharides, sugar alcohols and combinations thereof;
wherein the combination of components (a), (b), (c) and (d) constitutes at least 60 wt.% of the sealing powder;
wherein the sealing powder has a tapped density in the range of 0.3 - 0.9 g/ml;
wherein at least 90 wt.% of the powder has a diameter below 600 µm and not more than 10 wt.% of the powder has a diameter below 10 µm; and
wherein the components (a), (b), (c) and (d) may be contained in the same particles or in different particles.

2. Powder according to claim 1, wherein the electrophilic polymer is selected from an electrophilic polyoxazoline, electrophilic polyethylene glycol and combinations thereof.

3. Powder according to claim 1 or 2, wherein the nucleophilic cross-linker is selected from nucleophilic polyoxazoline, nucleophilic polyethylene glycol, polyethyleneimine and combinations thereof.

4. Powder according to any one of the preceding claims, wherein the water-insoluble polymer containing reactive nucleophilic groups is selected from protein, chitosan and combinations thereof.

5. Powder according to any one of the preceding claims, wherein the ratio between the total number of reactive electrophilic groups provided by the electrophilic polymer and the total number of reactive nucleophilic groups provided by the nucleophilic cross-linker lies in the range of 1: 0.05 to 1 : 0.4.

6. Powder according to any one of the preceding claims, wherein the powder comprises 20-80 wt.% of particles only containing components (a) and (d) and 20-80 wt% of particles only containing components (b), (c) and (d).

7. Powder according to claim 6, wherein the particles only containing components (a) and (d) and the particles only containing components (b), (c) and (d) together constitute at least 60 wt.% of the sealing powder.

8. Powder according to any one of claims 1-5, wherein the powder contains at least 60 wt.% of particles containing each of components (a), (b), (c) and (d).

9. Powder according to claim 8, wherein the powder contains at least 50 wt.% of the particles containing each of the components (a), (b), (c) and (d) in the form of agglomerates of sub-particles A only containing components (a) and (d) and sub-particles B only containing components (a), (c) and (d).

10. A method of preparing a bioresorbable sealing powder according to any one of the preceding claims comprising the steps of:
(a) providing particles A comprising the electrophilic polymer and the water-soluble dispersant;
(b) providing particles B comprising the nucleophilic cross-linker, the water-absorbing particles and the water-soluble dispersant;
(c) combining the particles A and particles B.

11. Method according to claim 10, wherein the particles A and the particles B are combined into agglomerates.

12. A device for applying a sealing powder comprising:
• a reservoir containing a bioresorbable sealing powder according to any one of claims 1-9;
• an elongated hollow tubular structure having a proximal and a distal end, the distal end having a powder outlet, the proximal end being connected to the reservoir,
• a manual air pump arranged to produce an air flow that carries powder from the reservoir via the elongated hollow tubular structure through the powder outlet.

13. A biocompatible, flexible, hemostatic sheet comprising:
• a cohesive fibrous carrier structure comprising a three-dimensional interconnected interstitial space; and
• the bioresorbable sealing powder according to any one of claims 1-9;
wherein the sealing powder is distributed within the interstitial space and/or fixated onto the fibrous carrier structure.

14. A kit of parts for preparing a bioresorbable sealing suspension comprising:
• a first container or compartment comprising a biocompatible liquid; and
• a second container or compartment comprising the bioresorbable sealing powder according to any one of claims 1-9.

15. A bioresorbable sealing suspension comprising:
• a biocompatible continuous liquid non-aqueous phase; and
• a dispersed phase comprising the bioresorbable sealing powder according to any one of claims 1-9.

## Patentansprüche

1. Bioresorbierbares Dichtungspulver, das Folgendes umfasst:
(a) mindestens 5 Gew.-% eines wasserlöslichen elektrophilen Polymers, das mindestens 3 reaktive elektrophile Gruppen trägt, die mit Aminogruppen unter der Bildung einer kovalenten Bindung reagieren können;
(b) 1-50 Gew.-% eines wasserlöslichen nukleophilen Vernetzers, der mindestens 2 reaktive nukleophile Gruppen trägt, die bei Vorhandensein von Wasser mit den reaktiven elektrophilen Gruppen des elektrophilen Polymers unter der Bildung einer kovalenten Bindung zwischen dem elektrophilen Polymer und dem nukleophilen Vernetzer reagieren können;
(c) 1-60 Gew.-% wasserabsorbierende Partikel, die mindestens 50 Gew.-%, berechnet auf das Gewicht der wasserabsorbierenden Partikel, eines wasserunlöslichen Polymers enthalten, das reaktive nukleophile Gruppen enthält, die aus Aminogruppen, Thiolgruppen und Kombinationen davon ausgewählt sind;
(d) 10-75 Gew.-% eines wasserlöslichen Dispersionsmittels, das bei 20 °C fest ist, wobei das besagte wasserlösliche Dispersionsmittel aus Monosacchariden, Disacchariden, Oligosacchariden, Zuckeralkoholen und Kombinationen davon ausgewählt ist;
wobei die Kombination der Komponenten (a), (b), (c) und (d) mindestens 60 Gew.-% des Dichtungspulvers ausmacht;
wobei das Dichtungspulver eine Stampfdichte im Bereich von 0,3 - 0,9 g/ml aufweist;
wobei mindestens 90 Gew.-% des Pulvers einen Durchmesser unter 600 µm aufweisen und nicht mehr als 10 Gew.-% des Pulvers einen Durchmesser unter 10 µm aufweisen; und
wobei die Komponenten (a), (b), (c) und (d) in denselben Partikeln oder in verschiedenen Partikeln enthalten sein können.

2. Pulver nach Anspruch 1, wobei das elektrophile Polymer aus einem elektrophilen Polyoxazolin, einem elektrophilen Polyethylenglykol und Kombinationen davon ausgewählt ist.

3. Pulver nach Anspruch 1 oder 2, wobei der nukleophile Vernetzer aus nukleophilem Polyoxazolin, nukleophilem Polyethylenglykol, Polyethylenimin und Kombinationen davon ausgewählt ist.

4. Pulver nach einem der vorhergehenden Ansprüche, wobei das wasserunlösliche Polymer, das reaktive nukleophile Gruppen enthält, aus Protein, Chitosan und Kombinationen davon ausgewählt ist.

5. Pulver nach einem der vorhergehenden Ansprüche, wobei das Verhältnis zwischen der Gesamtzahl der durch das elektrophile Polymer bereitgestellten reaktiven elektrophilen Gruppen und der Gesamtzahl der durch den nukleophilen Vernetzer bereitgestellten reaktiven nukleophilen Gruppen im Bereich von 1: 0,05 bis 1 : 0,4 liegt.

6. Pulver nach einem der vorhergehenden Ansprüche, wobei das Pulver 20-80 Gew.-% Partikel umfasst, die nur die Komponenten (a) und (d) enthalten, und 20-80 Gew.-% der Partikel, die nur die Komponenten (b), (c) und (d) enthalten.

7. Pulver nach Anspruch 6, wobei die Partikel, die nur die Komponenten (a) und (d) enthalten, und die Partikel, die nur die Komponenten (b), (c) und (d) enthalten, zusammen mindestens 60 Gew.-% des Dichtungspulvers ausmachen.

8. Pulver nach einem der Ansprüche 1-5, wobei das Pulver mindestens 60 Gew.-% Partikel enthält, die jeweils die Komponenten (a), (b), (c) und (d) enthalten.

9. Pulver nach Anspruch 8, wobei das Pulver mindestens 50 Gew.-% der Partikel, die jede der Komponenten (a), (b), (c) und (d) enthalten, in Form von Agglomeraten aus Unterpartikeln A, die nur die Komponenten (a) und (d) enthalten, und Unterpartikeln B, die nur die Komponenten (a), (c) und (d) enthalten.

10. Verfahren zum Herstellen eines bioresorbierbaren Dichtungspulvers nach einem der vorhergehenden Ansprüche, das die folgenden Schritte umfasst:
(a) Bereitstellen von Partikeln A, die das elektrophile Polymer und das wasserlösliche Dispersionsmittel umfassen;
(b) Bereitstellen von Partikeln B, die den nukleophilen Vernetzer, die wasserabsorbierenden Partikel und das wasserlösliche Dispersionsmittel umfassen;
(c) Kombinieren der Partikel A und Partikel B.

11. Verfahren nach Anspruch 10, wobei die Partikel A und die Partikel B zu Agglomeraten zusammengefasst werden.

12. Vorrichtung zum Aufbringen eines Dichtungspulvers, die Folgendes umfasst:
• ein Reservoir, das ein bioresorbierbares Dichtungspulver nach einem der Ansprüche 1-9 enthält;
• eine längliche hohle röhrenförmige Struktur mit einem proximalen und einem distalen Ende, wobei das distale Ende einen Pulverauslass aufweist und das proximale Ende mit dem Reservoir verbunden ist,
• eine manuelle Luftpumpe, die so angeordnet ist, dass sie einen Luftstrom erzeugt, der Pulver aus dem Reservoir über die längliche hohle röhrenförmige Struktur durch den Pulverauslass befördert.

13. Biokompatible, flexible, hämostatische Folie, die Folgendes umfasst:
• eine kohäsive faserige Trägerstruktur, die einen dreidimensionalen, miteinander verbundenen Zwischenraum umfasst; und
• das bioresorbierbare Dichtungspulver nach einem der Ansprüche 1-9;
wobei das Dichtungspulver in dem Zwischenraum verteilt und/oder auf der faserigen Trägerstruktur fixiert ist.

14. Teilesatz zum Herstellen einer bioresorbierbaren Dichtungssuspension, der Folgendes umfasst:
• einen ersten Behälter oder eine erste Kammer, der/die eine biokompatible Flüssigkeit enthält; und
• einen zweiten Behälter oder eine zweite Kammer, der/die das bioresorbierbare Dichtungspulver nach einem der Ansprüche 1-9 enthält.

15. Bioresorbierbare Dichtungssuspension, die Folgendes umfasst:
• eine biokompatible kontinuierliche flüssige nichtwässrige Phase; und
• eine dispergierte Phase, die das bioresorbierbare Dichtungspulver nach einem der Ansprüche 1-9 umfasst.

## Revendications

1. Poudre d'étanchéité biorésorbable comprenant
(a) au moins 5 % en poids de polymère électrophile hydrosoluble portant au moins 3 groupes électrophiles réactifs qui sont capables de réagir avec des groupes amine sous l'effet de la formation d'une liaison covalente ;
(b) 1 à 50 % en poids d'un agent de réticulation nucléophile hydrosoluble portant au moins 2 groupes nucléophiles réactifs qui, en présence d'eau, sont capables de réagir avec les groupes électrophiles réactifs du polymère électrophile sous l'effet de la formation d'une liaison covalente entre le polymère électrophile et l'agent de réticulation nucléophile ;
(c) 1 à 60 % en poids de particules absorbant l'eau contenant au moins 50 % en poids, calculé en poids des particules absorbant l'eau, de polymère insoluble dans l'eau contenant des groupes nucléophiles réactifs sélectionnés parmi des groupes amine, des groupes thiol et des combinaisons de ceux-ci ;
(d) 10 à 75 % en poids de dispersant hydrosoluble qui est solide à 20 °C, ledit dispersant hydrosoluble étant sélectionné parmi des monosaccharides, des disaccharides, des oligosaccharides, des alcools de sucre et des combinaisons de ceux-ci ;
où la combinaison des composants (a), (b), (c) et (d) constitue au moins 60 % en poids de la poudre d'étanchéité ;
où la poudre d'étanchéité a une densité tapée dans la plage de 0,3 à 0,9 g/ml ;
où au moins 90 % en poids de la poudre a un diamètre inférieur à 600 µm et pas plus de 10 % en poids de la poudre a un diamètre inférieur à 10 µm ; et
où les composants (a), (b), (c) et (d) peuvent être contenus dans les mêmes particules ou dans des particules différentes.

2. Poudre selon la revendication 1, où le polymère électrophile est sélectionné parmi une polyoxazoline électrophile, un polyéthylène glycol électrophile et des combinaisons de ceux-ci.

3. Poudre selon la revendication 1 ou 2, où l'agent de réticulation nucléophile est sélectionné parmi une polyoxazoline nucléophile, un polyéthylène glycol nucléophile, une polyéthylèneimine et des combinaisons de ceux-ci.

4. Poudre selon l'une quelconque des revendications précédentes, où le polymère insoluble dans l'eau contenant des groupes nucléophiles réactifs est sélectionné parmi des protéines, le chitosane et des combinaisons de ceux-ci.

5. Poudre selon l'une quelconque des revendications précédentes, où le rapport entre le nombre total de groupes électrophiles réactifs fournis par le polymère électrophile et le nombre total de groupes nucléophiles réactifs fournis par l'agent de réticulation nucléophile se situe dans la plage de 1:0,05 à 1:0,4.

6. Poudre selon l'une quelconque des revendications précédentes, où la poudre comprend 20 à 80 % en poids de particules contenant uniquement les composants (a) et (d) et 20 à 80 % en poids de particules contenant uniquement les composants (b), (c) et (d).

7. Poudre selon la revendication 6, où les particules contenant uniquement les composants (a) et (d) et les particules contenant uniquement les composants (b), (c) et (d) constituent ensemble au moins 60 % en poids de la poudre d'étanchéité.

8. Poudre selon l'une quelconque des revendications 1 à 5, où la poudre contient au moins 60 % en poids de particules contenant chacun des composants (a), (b), (c) et (d).

9. Poudre selon la revendication 8, où la poudre contient au moins 50 % en poids de particules contenant chacun des composants (a), (b), (c) et (d) sous la forme d'agglomérats de sous-particules A contenant uniquement les composants (a) et (d) et de sous-particules B contenant uniquement les composants (a), (c) et (d).

10. Procédé de préparation d'une poudre d'étanchéité biorésorbable selon l'une quelconque des revendications précédentes comprenant les étapes suivantes :
(a) fournir des particules A comprenant le polymère électrophile et le dispersant hydrosoluble ;
(b) fournir des particules B comprenant l'agent de réticulation nucléophile, les particules absorbant l'eau et le dispersant hydrosoluble ;
(c) combiner les particules A et les particules B.

11. Procédé selon la revendication 10, où les particules A et les particules B sont combinées en agglomérats.

12. Dispositif d'application d'une poudre d'étanchéité comprenant :
• un réservoir contenant une poudre d'étanchéité biorésorbable selon l'une quelconque des revendications 1 à 9 ;
• une structure tubulaire creuse allongée ayant une extrémité proximale et distale, l'extrémité distale ayant une sortie de poudre, l'extrémité proximale étant connectée au réservoir,
• une pompe à air manuelle agencée pour produire un flux d'air qui transporte de la poudre du réservoir via la structure tubulaire creuse allongée jusqu'à la sortie de poudre.

13. Feuille hémostatique flexible biocompatible comprenant :
• une structure porteuse fibreuse cohésive comprenant un espace interstitiel interconnecté tridimensionnel ; et
• la poudre d'étanchéité biorésorbable selon l'une quelconque des revendications 1 à 9 ;
où la poudre d'étanchéité est répartie dans l'espace interstitiel et/ou fixée sur la structure porteuse fibreuse.

14. Kit d'éléments pour préparer une suspension d'étanchéité biorésorbable comprenant :
• un premier récipient ou compartiment comprenant un liquide biocompatible ; et
• un deuxième récipient ou compartiment comprenant la poudre d'étanchéité biorésorbable selon l'une quelconque des revendications 1 à 9.

15. Suspension d'étanchéité biorésorbable comprenant :
• une phase liquide non aqueuse continue biocompatible ; et
• une phase dispersée comprenant la poudre d'étanchéité biorésorbable selon l'une quelconque des revendications 1 à 9.
